# EUROPEAN PATENT APPLICATION

(11) **EP 1 834 963 A1**
(43) Date of publication of application: **19.09.2007**
(21) Application number: 06111062.3
(22) Date of filing: 13.03.2006
(51) Int. Cl.: C07K 14/505, A61K 47/48, C07K 14/61, C07K 14/555, A61K 38/00, A61P 31/12

(54) **Di-polymer protein conjugates and processes for their preparation**

(71) Applicant: Siegfried Ltd., 4800 Zofingen (CH)
(72) Inventor: Bavand, Michael, 5000 Aarau (CH); Blasey, Horst, 4665 Oftringen (CH); Lang, Dietmar, 4054 Basel (CH)
(74) Representative: Huenges, Martin

(57) **Abstract**

The present invention refers to di-polymer protein conjugates and processes for their preparation. Especially, the present invention refers to di-polymer conjugates of erythropoietin, interferon and human growth hormone. Further, the present invention refers to the use of such di-polymer protein conjugate, especially di-pegylated protein conjugate, for the manufacture of a medicament for the treatment of disorders.

## Description

The present invention refers to di-polymer protein conjugates and processes for their preparation. Especially, the present invention refers to di-polymer conjugates of erythropoietin, interferon and human growth hormone. Further, the present invention refers to the use of such di-polymer protein conjugate, especially di-pegylated protein conjugate, for the manufacture of a medicament for the treatment of disorders.

Recombinant proteins in native form have been successfully used as therapeutics for over 20 years. However, the bioavailability of protein therapeutics is often limited by a short plasma half-life and susceptibility to protease degradation, which limits their maximum clinical potency.

In order to overcome these shortcomings, therapeutic proteins have been modified in order to increase their circulation half-life, e.g. by chemically or enzymatically coupling a polymer such as polyethylene glycol (PEG) to the protein. The modification of a protein by attaching polyethylene glycol moiety thereto is also known as pegylation. Proteins which were successfully modified by pegylation include erythropoietin (EPO), interferons such as interferon alpha, interferon beta and interferon gamma, granulocyte colony- stimulating factor (G-CSF or GCSF), interleukins such as IL-2 and IL-6, tumour necrosis factor (TNF), various cytokines and synthetic erythropoiesis protein (synthetic EPO).

Chemical modification of a protein by conjugation to polyethylene glycol results in improved thermal stability, reduced susceptibility to enzymatic degradation, shielding of the protein's antigenic determinants which leads to a reduced immunogenicity and a lower ability to react with antibodies, increased solubility, prolonged *in vivo* circulating plasma half-life due to decreased renal and cellular clearance of PEG-linked protein therapeutic (see Delgado et al., Crit Rev Ther Drug Carrier Syst 1992, 9(3,4): 249-304), lower peak plasma concentrations, smaller fluctuations in plasma concentrations, less frequent administration and therefore better quality of life. Thus, PEG-modified proteins are widely used for biomedical and biotechnological applications (see Inada et al., TIBTECH 1995, 13:86-91).
Methods for the attachment of polyethylene glycol moieties to proteins are described in Pasut et al., Expert Opin. Ther. Patents 2004, 14(6): 859-894; W.P. Sheffield, Curr. Drug Targets 2001, 1-22; F.M Veronese, Biomaterials 2001, 22:405-417; and S. Zalipsky, Adv. Drug Deliv Rev. 1995, 16:157-182 and methods for the attachment of polyethylene glycol moieties to proteinoids are described in G. Kochendoerfer, Expert Opin Biol Ther. 2003, 8, 1253-61.

In general, polyethylene glycol molecules are coupled to the protein via a functional group of the protein. Each functional group in the amino acid chain of the protein, which is nucleophilic, i.e. has electron donor ability, may react with a complementary group being attached to a PEG molecule. Such nucleophilic groups include α-amino, ε-amino, carboxyl, thiol, hydroxyl, imidazole, and guanidine groups. For example, the amino groups of a protein, i.e. the a-amino group (N-terminus) and e-amino groups of lysine residues, are used as the reactive or functional groups for PEG coupling (see F.M. Veronese, Biomaterials 2001, 22: 405-417).

US 4,179,337 discloses PEG attachment to a protein via an acylation reaction resulting in an amide linkage between PEG and the protein.

US 4,002,531 describes that reductive alkylation was used for PEG attachment to an enzyme resulting in an amine linkage between PEG and protein.

Further, the coupling of PEG to amino groups of a protein was described in EP 0 067 029 (acylation of he moglobin), EP 0 098 110 (acylation of urokinase, kallikrein or leukocyte interferon), EP 0 154 316 (reductive alkylation of lymphokines at neutral pH), EP 0 154 432 (acylation of urokinase), EP 0 229 108 (acylation of interferon beta, interleukin-2 and immunotoxins), EP 0 247 860 (acylation of tumour necrosis factor), EP 0 335 423 (acylation of G-CSF), EP 0 372 752 (alkylation of CD4 immunoadhesin at neutral pH), EP 0 439 508 (alkylation of, inter alia, GM-CSF or G-SCF with tresylchloride), EP 0 442 724 (acylation of interleukin 6), EP 0 473 268 (acylation of calcitonin, interleukin-2 or granulocyte colony-stimulating factor) and EP 0 539 167 (acylation of proteins). However, the pegylation reactions described in these patent applications stated above result in multi-pegylated products, i.e. protein conjugates having more than one PEG moiety per protein. With a growing number of PEG moieties, multi-pegylated protein conjugates are increasingly difficult to characterize, which hampers the regulatory approval of a therapeutic protein as a medicinal product.

Hence, keeping the number of PEG moieties in a therapeutic protein low facilitates the generation of a defined product with a predictable activity and a lot-to-lot product consistency which makes it useful for pharmaceutical preparations. Further, a low number of PEG moieties per protein reduces the risk that the receptor recognition in enzyme- substrate interactions is impaired. Thus, pegylated proteins for biopharmaceutical applications preferably have a low number of PEG units per protein.

This lead to the development of PEG protein conjugates with a low number of PEG units per protein, as described in the following.

WO 96/11953 describes methods for attaching one polyethylene glycol molecule to the α-amino group (N-terminus) of a polypeptide under conditions of a reductive alkylation and at a pH sufficiently acidic to selectively activate the a-amino group. The difference in pKa values between the a-amino group at the N-terminus, the pKa of which ranges from pH 6.8 to 8.2, and e-amino groups of lysyl side chains, the pKa of which ranges from pH 10.4 to 10.5 (see Azarkan et al., Rec Res Develop Biotech Bioeng 1999, 2, 77-93) suggests that pegylation in an acidic environment selectively yields N-terminal mono-pegylation of proteins.

Compared to mono-pegylated proteins, di-pegylated proteins, however, have some significant advantages:
- The maximum number of ethylene oxide units per linear PEG molecule limits the molecular weight to approximately 30 kDa (see Harris and Chess, Nat. Rev. Drug Discovery 2003, 2(3), 214-223). Attaching a second PEG molecule therefore allows increasing the molecular weight of the PEG portion in the PEG protein conjugate.
- PEG molecules with high molecular weight lead to increased viscosity of the pegylated protein solution. Attaching two PEG molecules of lower molecular weight instead of one high molecular weight PEG molecule can reduce this effect.
- In case of equivalent total molecular weight of the PEG-protein conjugate, the attachment of two instead of one PEG molecules yields an increased hydrodynamic radius of the PEG protein conjugate, which leads to protracted clearance and, thus, extends the duration of biological activity of the conjugate.
- A more effective shielding of epitopes of the protein can be achieved by linkage of the protein with a second PEG molecule, so that proteolytic sensitivity and immunogenicity are reduced, while the stability of the PEG-protein conjugate is improved. This is particularly advantageous for proteinoids and non-recombinantly synthesised protein fragments that are joined via non-natural linkers, which may potentially increase immunogenicity.
- Attachment of a second PEG moiety increases the hydrophilic character of the PEG protein conjugate. Hence, the solubility is improved in a buffer system, which implies reduced risk of conjugate aggregation.

In view of these advantages, attempts were made to synthesise predominantly di-pegylated protein conjugates. These attempts included genetic manipulation of the native protein sequence, which provides a mean for site-directed PEG protein coupling as a prerequisite to generate di-pegylated protein mutein conjugates.

EP 0 730 470 relates to a process for preparing conjugates of IFN alpha in the presence of a surfactant at a pH from pH 7 to pH 9 in order to obtain preferential attachments at lysine residues outside the active site region of IFN alpha.

EP 0 809 996 and EP 1 055 685 independently disclose the conjugation of IFN alpha with branched PEG molecules by means of acylation.

EP 0 605 963 reports on EPO being conjugated with water-soluble polymers (e.g., PEG) having a hydrazide or oxylamine moiety capable of covalent bonding through chemical reaction with an oxidizable functional group such as polyol, lactol, amine, carboxylic acid or a carboxylic acid derivative on the proteins or glycoproteins. According to this report, a water-soluble polymer, such as PEG, can be attached to various functional groups on amino acids or sugar residues constituting a protein or glycoprotein to yield a coupling product with several, e.g. 6 to 34, PEG molecules (molecular weight: 2000 to 12000) per protein molecule. In such a PEG-conjugated protein or glycoprotein with many PEG molecules, it is difficult to control the positions and number of PEG molecules attached to the protein or glycoprotein and it is hard to obtain an uniform PEG-conjugated protein or glycoprotein.

Further examples for unspecific PEG attachment are described in EP 1 196 443 and EP 1 135 493 resulting in the formation of mono- to sixta-pegylated EPO conjugates being obtained by coupling PEG moieties to the protein via acylation or reductive alkylation. Possible PEG attachment sites according to EP 1 196 443 are amide linkages to alpha- and epsilon amino groups, and according to EP 1 135 493 are amine linkages to alpha- and epsilon amino groups.
Although, non- glycosylated EPO was modified by reductive alkylation in EP 1 135 493, no specific reaction conditions and no direction is provided in the patent to a person skilled in the art as leading to the pegylation sites of EPO or to the preferable sites for modification.

US 4,179,337 discloses methods of PEGylating enzymes and hormones to obtain physiologically active non-immunogenic, water- soluble polypeptide conjugates. hGH is mentioned as one example of a hormone to bePEGylated.

Clark et al. (J. Biol. Chem. (1996) 271(36): 21969-21977 discloses unspecific PEG attachment to hGH by acylation with PEG-NHS.

EP 0 458 064 discloses PEG attachment to introduced or naturally present cysteine residues in somatotropin.

WO 95/11987 suggests the attachment of PEG to the thio group of a cysteine residue being either present in the parent molecule or introduced by site directed mutagenesis. WO 9511987 relates to PEGylation of proteasenexin-1, however PEGylation in general of hGH and other proteins is suggested as well.

WO 99/03887 discloses, e.g., growth hormone modified by insertion of additional cysteine residues and attachment of PEG to the introduced cysteine residues.

WO 00/42175 relates to a method for making proteins containing free cysteine residues for attachment of PEG. WO 0042175 discloses hGH muteins

Further hHG muteins are described in WO 97/11178 (as well as US 5849535, US6004931, and US 6022711), which relates to the use of GH variants as agonists or antagonists of hGH. WO 97/11178 also discloses pegylation of hGH, including PEG attachment to lysine residues and the introduction or replacement of lysine residues (e. g. K168A and K172R) to lower the number of potential attachment sites.

WO 2005075021 describes the chemical modification of hGH muteins, a hGH analogue with several mutations in the receptor binding regions to allow an improved binding affinity for hGH cellular receptor. The genetically engineered hGH mutein conjugate has decreased PEG attachment sites heterogeneity because of attaching a single polyethylene glycol moiety to the N-terminus.

A similar approach is disclosed in EP 1 591 467, which describes different chemical structures of differently defined PEG molecules, especially PEG aldehydes, being site-specific conjugated to proteins, such as IFN alpha, EPO, hGH, at the N-terminus.

Finally, WO 02/055532 describes genetically engineered hGH muteins having at least one non-polypeptide moiety covalently attached. Especially hGH muteins with introduced cysteine residue, but not limited to, are used for pegylation

In such PEG protein conjugates, it is difficult to control the positions and number of PEG molecules attached to the protein or glycoprotein and it is hard to obtain a uniform PEG-conjugated protein or glycoprotein.

This leads to the development of PEG protein conjugates with a low number of PEG units per protein, as described in the following.

WO 96/11953 and EP 0 822 199 describe a method for attaching one polyethylene glycol molecule to the α-amino group (N-terminus) of a polypeptide, such as IFN gamma, under conditions of a reductive alkylation and at a pH sufficiently acidic to selectively activate the alpha-amino group. The difference in pKa values between the alpha-amino group at the N-terminus, of which the pKa ranges from pH 6.8 to 8.2, and epsilon-amino groups of lysyl side chains, of which the pKa range from pH 10.4 to 10.5 (see Azarkan et al., Rec Res Develop Biotech Bioeng 1999, 2, 77-93) suggests that pegylation in an acidic environment selectively yields N-terminal mono-pegylation of proteins.

EP 1 507 755 (WO 03/049699) and Chamow et al. (Bioconjugate Chemistry (1994) 5(2): 133-140) describe that pegylation of proteins at pH 7.5, which is in the vicinity of neutrality, still leads (i) to mainly mono-pegylated protein conjugates having the PEG attachment at the alpha amino group and (ii) that the remaining PEG molecules are evenly attached throughout the molecule because of the indistinguishable reactivity of the epsilon amino groups at neutral pH.

Its application to produce mono-pegylated EPO derivatives having the PEG attachment at the N-terminus is described in EP 1 267 942. Alternatively using a different coupling chemistry, acylation via active PEG esters, mono-pegylated EPO derivatives having one PEG molecule attached to an epsilon amino group are reported in EP 1 333 036. Preferred attachment occurred to the amino acid residue, Lysine 52.

EP 1 144 613 describes EPO mutein conjugates, wherein the EPO amino acid sequence differs from the amino sequence of human EPO in at least one added free cysteine amino acid residue which comprises an attachment group for PEG. In particular, EP 1 144 613 describes a mono-pegylated EPO mutant that is pegylated at specifically introduced amino groups by acylation. According to EP 1 144 613 it is preferred to introduce at least one cysteine residue in EPO in order to allow a site-specific pegylation by acylation.

WO 2004/009627 discloses EPO mutein conjugates, wherein one or more of the lysine residues available for pegylation is replaced with an amino acid that cannot be pegylated to allow a site specific pegylation.

Muteins, apart from the disadvantageous complexity of their development, bear additional risks such as potential immunogenicity caused by amino acid changes of the natural sequence. In contrast to the di-pegylation processes described in WO 00/21574 and WO 01/51510, it would therefore be desirable to di-pegylate a recombinant or natural protein having a wild-type amino acid sequence without the need to mutate the protein for the purpose of pegylation.

An example is given in WO 00/44785, which describes an unspecified di-pegylated G-CSF conjugate that is obtained by coupling PEG moieties to the protein via acylation. Possible PEG attachment sites according to WO 00/44785 are amide linkages to a- and e-amino groups and ester-linkages to primary hydroxyl groups. However, the pegylation reaction did not yield specifically dipegylated G-CSF molecules.

Although acylation allows to di-pegylate G-CSF, PEG-protein conjugates synthesized by acylation have a lower stability and show increased aggregation (see Kinstler et al., Pharm. Res. 1996, 13:996-1002).

Similar problems did occur when it was tried to conjugate other commercially interesting molecules such as erythropoietin, interferons or human growth hormone with polymers. Often the pegylation reaction resulted in unspecific polymer attachment. In some cases, muteins of the above-mentioned molecules were used to avoid or reduce unspecific polymer attachment.

In view of the above-mentioned problems, there is a need for stable and defined polymer-protein conjugates with two polymer units per protein.

It is thus an object of the present invention to provide stable and defined polymer-protein conjugates with two polymer units per protein. In particular, it is an object of the present invention to provide stable and defined di-pegylated protein conjugates.

It is a further object of the present invention to provide polymer-protein conjugates with two polymer units per protein, in particular di-pegylated protein conjugates, wherein the protein has the wild-type or naturally occurring amino acid sequence or is a synthetic protein-like mimic of the wild-type protein, i.e. a proteinoid, having at least the biological activity of the wild-type protein.

Another object of the present invention is to provide polymer-protein conjugates with two polymer units per protein, in particular di-pegylated protein conjugates, which have a longer circulating half-time and a greater *in vivo* biological activity than the corresponding unconjugated protein.

It is another object of the present invention to provide a method for significantly increasing the yield of polymer-protein conjugates with two polymer units per protein, in particular dipegylated protein conjugates.

Further, it is an object of the present invention to provide a method for predominantly preparing a polymer-protein conjugate with two polymer units per protein in high yields, which shows good product consistency and easily available educts.

Finally, it is an object of the present invention to identify conditions for a process for the preparation of a polymer-protein conjugate with two polymer units per protein, in particular a di-pegylated protein conjugate under which two pre-selected attachment sites of the protein are predominantly conjugated with a polymer unit.

According to the invention, these and other objects are attained by the feature combinations of the independent claims. Advantages and embodiments of the invention are defined in the dependent claims.

It has been found that stable and biological active polymer-protein conjugates with two polymer units per protein, in particular PEG protein conjugates can be provided, wherein two nitrogen atoms of amino groups of the protein are each conjugated with one polymer unit, in particular a polyethylene glycol moiety.

In particular, polymer conjugates of erythropoietin, interferon and human growth hormone with two polymer units are provided.

In a further aspect of the present invention, a pharmaceutical preparation is provided which comprises at least one, and preferably two di-polymer-protein conjugates according to the present invention.

In a further aspect of the present invention, a process for the preparation of a polymer-protein conjugate is provided, which comprises reacting a protein having at least two amino groups with a polymer reagent having a single aldehyde group in the presence of a reducing agent wherein the reaction time is chosen such that polymer-protein conjugates with two nitrogen atoms of amino groups of the protein being conjugated with a polymer unit via an amine linkage are preferably prepared.

Further, the present invention relates to the use of a polymer-protein conjugate such as a polyethylene glycol-protein conjugate according to the present invention for the manufacture of a medicament for the treatment of a disorder. The kind of disorder to be treated depends on the kind of protein conjugated to the polymer. For example, the disorder might be characterized by reduced haematopoietic or immune function. If the protein is erythropoietin, the disorder is usually a type of anemia, for example renal anemia. If the protein is growth hormone (also called somatotropin), the disorder is usually growth hormone deficiency. If the protein is an interferon, the disorder might be an infectious disease.

In particular, the invention is based on the finding that preferably two polymer units, in particular polyethylene glycol molecules, can be attached to a protein under conditions of a reductive alkylation by selecting a suitable pH, e.g. a pH about 7.

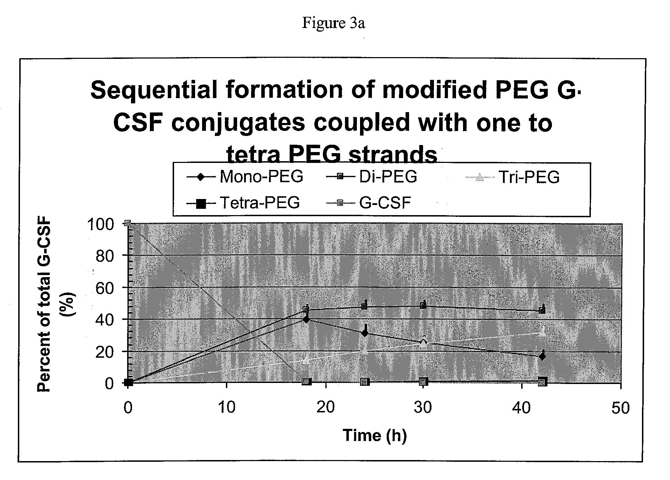

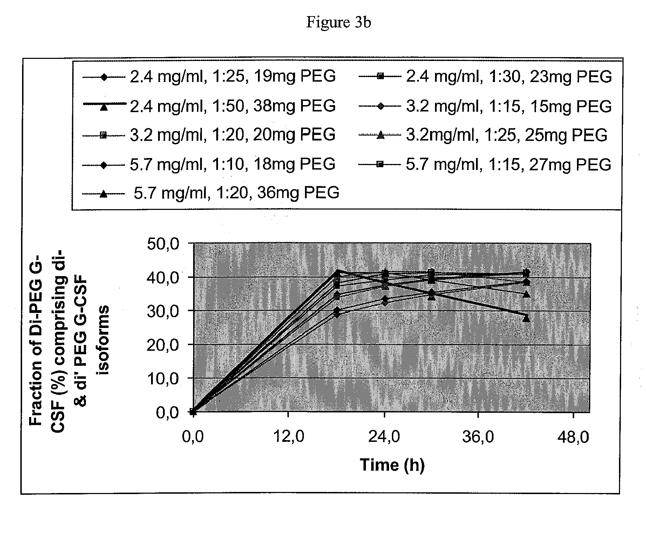

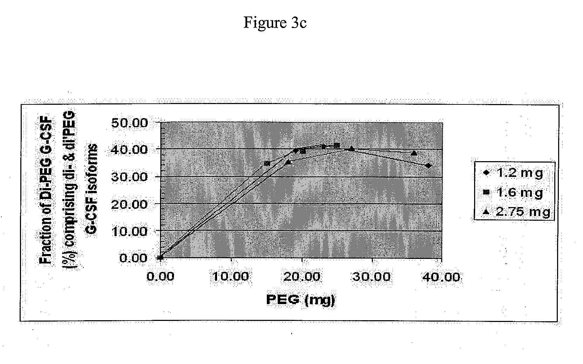

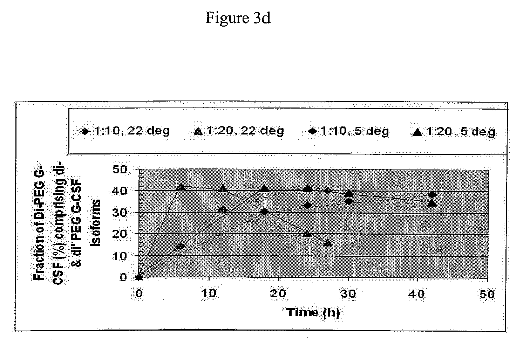

Further, it has been found, that the reaction time is a crucial parameter for the formation of polymer-protein conjugates with two polymer units per protein, in particular di-pegylated protein conjugates according to the present invention. E.g., it has been found that the formation of pegylated protein conjugates according to the invention occurs sequentially in a time-dependent order from mono-, di-, tri- to higher pegylated protein conjugates (see Figure 3).

Further, it has been found, that the molar ratio of protein to polymer and/or the stoichiometric ratio of the number of amino groups within a protein to the number of polymer molecules is crucial for the formation of polymer-protein conjugates with two polymer units per protein, in particular di-pegylated protein conjugates according to the present invention. Further, it has been found that, when compared to non-glycosylated proteins, a lower stoichiometric ratio of polymer moelcules to the amino groups within the protein is required for the formation of polymer-protein conjugates with two polymer units per protein, in particular di-pegylated protein conjugates according to the present invention if the protein is glycosylated.

Further, it has been found that the process yield could be significantly increased by introducing a recycling step in which non-desired reaction products representing non-reacted or partially reacted protein molecules are applied to a second polymer attachment process step such as a second pegylation reaction step yielding further di-pegylated protein conjugate and thus contributing to a higher overall process yield of di-pegylated protein conjugate. Unexpectedly, the recycling step results in a prominent yield of di-pegylated protein conjugate with high quality, being comparable to the quality obtained in the first pegylation reaction. This principle of recycling non-desired reaction products to obtain a higher yield of polymer-protein conjugates with two polymer units per protein is also applicable to other polymer protein conjugate forming reactions.

Further, it has been found that the method of protein modification according to the present invention allows one to preferably or predominantly attach polymer units at a protein at specific sites of the protein taking advantage of a different reactivity of amino groups of the same type (e.g. ε-amino group of lysine residues) depending on the amino acid residues adjacent to the amino acid with the reactive amino group in the sequence of the protein. Thus, the process according to the present invention is preferably performed at a pH, which takes advantage of the pKa difference among e.g. different ε-amino groups, different guanidino groups and/or different imidazole groups of the protein. This leads to a substantially homogenous preparation of di-polymer protein conjugates having the polymer predominantly attached to defined or pre-selectable or pre-selected or predetermined amino groups of the protein.

"Substantially homogenous" within the context of this invention is intended to mean that the preparation contains mainly di-polymer protein conjugates having the polymer predominantly attached to defined or pre-selectable or pre-selected or predetermined amino groups of the protein and to a lesser extent unconjugated protein species or proteins having attached one or more than two polymers or proteins having the polymer attached to other amino groups than the preselected ones. Preferably, the preparation contains at least 50%, more preferably at least 60, 65, 70 or 75%, even more preferably at least 80, 85 or 90 % and most preferably at least 92, 94, 96, 98 or 99% di-polymer protein conjugates having the polymer attached to the preselected amino groups, in relation to the total amount of proteins present in the preparation.

Finally, it has been found that a homogenous preparation of di-polymer protein conjugate comprising either two di-polymer protein isoforms in a defined mixture or a single di-polymer protein isoform show a biological activity which is at least comparable to a similar monopegylated protein and even better than the corresponding non-pegylated protein.

Preferably, the polymer-protein conjugates according to the present invention are prepared by reductive alkylation. Thus, the present invention preferably provides polymer-protein conjugates, wherein two nitrogen atoms of amino groups of the protein are each conjugated with a polymer unit via an amine linkage.

In the context of the present invention, the term amino group includes primary and secondary amino groups and in particular NH- or NH₂-groups in side-chains of amino acids such as NH₂-groups in the side-chain of lysine, NH- or NH₂-groups in the guanidino group of arginine or NH-groups in the imidazole side-chain of histidine. In particular, the term amino group includes α- (N-terminal) amino groups and ε-amino groups which are NH₂-groups in the side-chain of lysine.

The polymer unit preferably comprises at least one polymer moiety and a linker moiety, which is between at least the one polymer moiety and the amine linkage. The linker moiety may be linear or branched. If the linker moiety is branched, a polymer unit may comprise more than one polymer moiety.

The linker moiety is preferably an aliphatic linker moiety. Suitable aliphatic linker moieties also include substituted alkyl diamines and triamines, lysine esters and malonic ester derivatives. The linker moieties are preferably non-planar, so that the polymer chains are not rigidly fixed. Preferably, the linker moiety includes a multiple-functionalized alkyl group containing up to 18, and more preferably from 1 to 10 carbon atoms. A hetero-atom such as nitrogen, oxygen or sulfur may also be included within the alkyl chain. The linker moiety may be branched, for example at a carbon or nitrogen atom. Examples for branched linker moieties and the resulting branched polymer units as well as methods for their preparation are described in WO 95/11924 and WO 03/049699, which are herein incorporated by reference. "Branched" within the meaning of the present invention is intended to comprise also combed moieties.

In a preferred embodiment of the present invention, the linker moiety comprises at least one methylene group attached to the nitrogen atom of the amine linkage, e.g. from 1 to 12, preferably from 1 to 5, more preferably from 1 to 3 and most preferably two methylene groups which are directly attached to the nitrogen atom of the amine linkage.

One preferred polymer-protein conjugate according to the present invention has the formula:

[(R-L¹-Polymer)_{y}-L²-(CH₂-)ₘ-NH]₂-P

wherein R is H, lower alkyl, aryl or any suitable protecting group; Polymer is a polymer which is suitable to be conjugated with proteins; m is an integer representing the number of methylene groups, P is a biologically active protein or proteinoid wherein two nitrogen atoms of amino groups of the protein or proteinoid (represented by NH in the above formula) are each conjugated with a polymer unit, L¹ is O, N, S and/or a branched or non-branched linker moiety which can be absent or present; L² is a branched or non-branched linker moiety which can be absent or present; and y is a integer with the proviso that y is 1 in the absence of L² and y is at least 1 in the presence of L².

R is preferably a lower alkyl or an aryl such as benzyl. The term lower alkyl includes lower alkyl groups containing e.g. from 1 to 7 carbon atoms, preferably from 1 to 4 carbon atoms, such as methyl, ethyl, propyl, isopropyl, butyl, etc., with methyl being particularly preferred.

The linker L¹ which is part of the linkage between at least one polymer moiety and the suitable protecting group and/or the linker L² which is part of the linkage between the at least one polymer moiety, such as ethylene oxide residues of a PEG chain, and the nitrogen atom of the protein or proteinoid and, in particular, between the ethylene oxide residues of the PEG chain and the methylene groups, can independently be absent or present and are independently selected from a branched or non-branched linker moiety having any arbitrary structural formulae, such as the linker moieties described above, e.g. (-CO)₂-CH-, -CO-O-, and/or -CO-NH-. The linker L¹ might also be O, N and/or S.

Usually, the number m of methylene groups forming a part of the linkage between polymer moiety, such as the ethylene oxide residues of a PEG chain, and the nitrogen atom of the protein or proteinoid, to which the polymer moiety such as a PEG reagent is attached to, is in a range from 1 to 12, preferably from 1 to 5, more preferably from 1 to 3 and is most preferably 2.

Usually, the number y, in the presence of L², is in a range from 1 to 10, preferably from 1 to 5, more preferably from 1 to 3 and is most preferably 2.

The polymer moiety is usually a substantially non-antigenic or non-immunogenic polymer chain. Further, the polymer moieties used are preferably selected from among water-soluble polymer moieties. This has the advantage that the protein to which the water-soluble polymer moieties are attached or conjugated does not precipitate in an aqueous environment such as a physiological environment. For reductive alkylation, the polymer selected should further have a single reactive aldehyde, so that the degree of polymerization may be controlled as provided for in the present processes. The polymer unit as well as the polymer moiety may be branched or unbranched. Preferably, for therapeutic use of the end-product preparation, the polymer will be pharmaceutically acceptable. One skilled in the art will be able to select the desired polymer moiety based on considerations such as whether the polymer-protein conjugate will be used therapeutically, and if so, the desired dosage, circulation time, resistance to proteolysis and other considerations.

Preferably, the polymer moiety may be selected from the group consisting of polyalkylene glycol moieties, polysaccharide moieties such as dextran and its derivatives, polysaccharide and its derivatives, pyrrolidone moieties such as polyvinyl pyrolidone, cellulose moieties such as carboxymethyl cellulose, polyvinyl alcohol, poly-1,3-dioxolan, poly-1,3,6-trioxane, ethylene-maleic anhydride copolymer, polyaminoacid moieties and/or polyacrylamide moieties and/or other similar non- immunogenic polymer moieties (either homopolymers or random copolymers) and/or derivatives thereof. Such polymers are also capable of being functionalized or activated for inclusion in the present invention.

In particular, the polymer moiety is a polyalkylene glycol moiety. The term polyalkylene glycol designates polyalkylene glycol radicals or polyalkylene glycol moieties, where the alkylene radical is a straight or branched chain radical. The term polyalkylene glycol also comprises polyalkylene glycols formed from mixed alkylene glycols such as polymers containing a mixture of polyethylene and polypropylene radicals and polymers containing a mixture of polyisopropylene, polyethylene and polyisobutylene radicals. Preferably, a polyalkylene glycol moiety in the polymer protein conjugates according to the present invention is a polyethylene glycol moiety or polyethylene glycol residue formed by removal of the two terminal hydroxyl groups.

Within this group are a-substituted polyalkylene oxide derivatives such as methoxy polyethylene glycols (mPEG) or other suitable alkyl substituted polyalkylene oxide derivatives such as those containing mono- or bis-terminal C₁-C₄ groups. Straight-chained non-antigenic polymers such as monomethyl polyethylene glycol homopolymers are preferred. Alternative polyalkylene oxides such as other polyethylene glycol homopolymers, polyethylene glycol heteropolymers, other alkyl polyalkylene oxide block copolymers and copolymers of block copolymers of polyalkylene oxides are also useful.

The foregoing is merely illustrative and not intended to limit the type of non-antigenic polymers or polymer moieties suitable for use herein.

An especially preferred polymer according to the present invention is polyethylene glycol resulting in PEG-protein conjugate of the formula:

[(R-L¹-PEG_{y})-L²-(CH₂)ₘ-NH]₂-P

wherein R, P, L¹, L², y, m are defined as described above.

In particular, PEG has the formula (CH₂-CH₂-O)ₙ with n representing the number of ethylene oxide residues in a polyethylene glycol unit. Further, it is preferred, that L¹ is O, L² is absent and y is 1.

The nitrogen atom of the amino group to which a polymer unit may be attached according to the present invention is preferably selected from the group consisting of nitrogen atoms of the α-amino group, ε-amino groups, guanidinium groups and imidazole groups. It is preferred, but not limited, to attach a polymer unit to the nitrogen atoms of a primary amino group.

The biologically active protein, which can be conjugated in accordance with this invention, may be any of the conventional therapeutic proteins. Further, in the context of the present invention the term protein is understood to include proteins and proteinoids. In the context of the present invention, proteinoids are synthetic protein-like molecules which have the identical amino acid sequence as the corresponding native protein, but are chemically synthesised in the form of peptides which are linked together to form the native protein- like structure with a resulting biological activity similar to the native protein. These proteinoids are potential protein therapeutics.

Proteins which can be conjugated with two polymer units and especially be di-pegylated according to the present invention include non- mutated and mutated proteins such as, but not limited to, growth factors, antibodies, hormones, in particular therapeutically active proteins such as, but not limited to, erythropoietin, interferon alpha, interferon beta, interferon gamma, consensus interferon, GM-CSF, hemoglobin, hormones such as growth hormone, interleukins such as interleukin-2 and interleukin-6, tumour necrosis factor, various cytokines as well as immunoglobulins such as IgG, IgE, IgM, IgA, IgD and/or structural and/or functional variants and/or fragments and/or analogues thereof as well as their proteinoids or synthetic protein- like forms such as synthetic erythropoiesis protein (synthetic EPO). Erythropoietin, interferon and human growth hormone are particularly preferred proteins according to the present invention.

Within the scope of this invention, analogues of wild-type proteins preferably do not comprise additional polymer attachment sites such as additional amino groups as compared to the wild-type sequence. Moreover, analogues preferably do not comprise mutations of the amino acids containing amino groups naturally present in the wild-type amino acid sequence. However, the use of amino acid sequences of the wild-type protein are preferred.

The proteins which can be conjugated with two polymer units may be glycosylated or non-glycosylated. For example, it has been shown for erythropoietin that its activity greatly depends on its glycosylation. Naturally occurring erythropoietin is both N- and O-glycosylated at positions 24, 38 and 83 and 126 of the mature protein, respectively. Glycosylated proteins which are to be used for the conjugation according to the present invention preferably have the same glycosylation pattern as the naturally occurring proteins. However, the present invention also encompasses glycosylated proteins having a different glycosylation pattern than the naturally occurring proteins, but which have essentially the same or even a higher biological activity than the naturally occurring proteins. The term "glycosylated protein" is intended to mean that the protein has at least one carbohydrate molecule attached to amino acid chain.

In general, proteins useful in the practice of this invention, such as erythropoietin, IFN α 2a and hGH, may be of any form isolated from mammalian organisms, a product of prokaryotic or eukaryotic host expression of exogenous DNA sequences obtained by genomic or cDNA cloning or by DNA synthesis or alternatively a product of chemical synthetic procedures or by endogenous gene activation. Thus, the protein can be of a natural or recombinant source obtained from tissue, mammalian or microbial cell cultures, plant cell cultures, transgenic animals, yeasts, fungi and/or transgenic plants. Suitable prokaryotic hosts include various bacteria such as *E. coli;* suitable eukaryotic hosts include yeasts such as **S.** *cerevisiae or Pichia pastoris,* mammalian cells such as chinese hamster ovary cells or monkey cells, transgenic animals such as mice, rabbit, goat, sheep, plant cell culture and transgenic plants such as *Physcomitrella patens* (a moss). Depending upon the host employed, the protein expression product may be glycosylated with mammalian, plant or other eukaryotic carbohydrates, or it may be non-glycosylated.

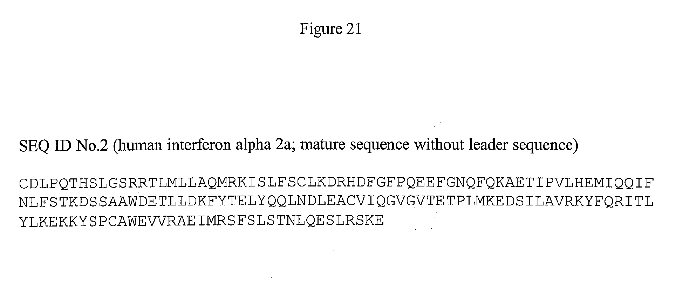

According to one preferred embodiment, the protein is a protein of the interferon family. Preferably, the protein is an interferon α protein, most preferably it is interferon a 2a. If the protein is interferon a 2a, it has preferably the sequence according to SEQ ID No. 2 (see Figure 21). The present invention contemplates the use of any and all such forms of interferon a 2a, also for example biologically functional analogues of the protein. These analogues may include those having amino acid additions, deletions and/or substitutions as compared to the interferon a 2a amino acid sequence according to SEQ ID No. 2. Within the scope of this invention, these analogues do not comprise additional polymer attachment sites such as additional amino groups as compared to the interferon a 2a amino acid sequence according to SEQ ID No. 2. Moreover, the analogues do not comprise mutations of the amino acids containing amino groups naturally present in the interferon a 2a amino acid sequence according to SEQ ID No. 2. However, the use of the amino acid sequence of the wild-type protein according to SEQ ID No. 2 is preferred.

If the protein is one having the activity of interferon a 2a, the two amino groups for polymer attachment are preferably selected from the group consisting of the α-amino group (N-terminus) and e-amino groups of lysine residues of interferon a 2a. It has been shown that among the potential PEG attachment sites the lysine residues at positions 121, 131, 134, 31 and 49 of the mature protein according to SEQ ID No. 2 are most preferred, because they are accessible to solvent. However, the present invention also relates to the conjugation of polymer groups such as PEG to the ε-amino group of lysine 70, 83, 112, 164, 23 and/or lysine 133 of the mature protein sequence.

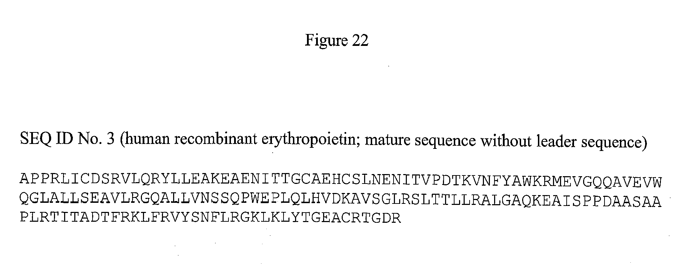

According to a further preferred embodiment, the protein is glycosylated or non- glycosylated erythropoietin. If the protein is erythropoietin, it has preferably the sequence according to SEQ ID No. 3 (see Figure 22). The present invention contemplates the use of any and all such forms of erythropoietin, also for example biologically functional analogues of the protein. The present invention further contemplates the use of both glycosylated and non- glycosylated erythropoietin. These analogues may include those having amino acid additions, deletions and/or substitutions as compared to the erythropoietin amino acid sequence according to SEQ ID No. 3. Within the scope of this invention, these analogues do not comprise additional polymer attachment sites such as additional amino groups as compared to erythropoietin amino acid sequence according to SEQ ID No. 3. Moreover, the analogues do not comprise mutations of the amino acids containing amino groups naturally present in the erythropoietin amino acid sequence according to SEQ ID No. 3. However, the use of the amino acid sequence of the wild-type protein according to SEQ ID No. 3 is preferred.

If the protein is one having the activity of erythropoietin, the two amino groups for polymer attachment are preferably selected from the group consisting of the α-amino group (N-terminus) and e-amino groups of lysine residues of erythropoietin. It has been shown that among the potential PEG attachment sites lysine residues at positions 52, 116 and 152 of the mature protein according to SEQ ID No. 3 are most preferred, because they are accessible to solvent. However, the present invention also relates to the conjugation of polymer groups such as PEG to the ε-amino group of lysine 20, 45, 97, 140 and/or lysine 154 of the mature protein sequence.

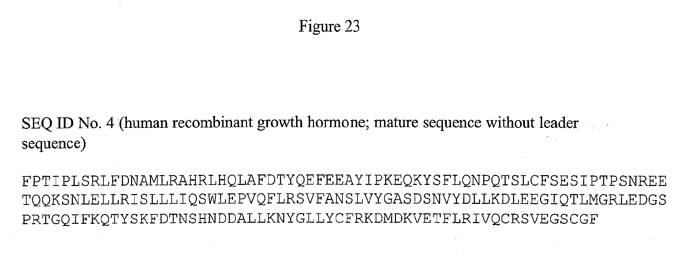

According to a further preferred embodiment, the protein is human growth hormone (hGH). If the protein is hGH, it has preferably the sequence according to SEQ ID No. 4 (see Figure 23). The present invention contemplates the use of any and all such forms of hGH, also for example biologically functional analogues of the protein. These analogues may include those having amino acid additions, deletions and/or substitutions as compared to the hGH amino acid sequence according to SEQ ID No. 4. Within the scope of this invention, these analogues do not comprise additional polymer attachment sites such as additional amino groups as compared to the hGH amino acid sequence according to SEQ ID No. 4. Moreover, the analogues do not comprise mutations of the amino acids containing amino groups naturally present in the hGH amino acid sequence according to SEQ ID No. 4. However, the use of the amino acid sequence of the wild-type protein according to SEQ ID No. 4 is preferred.

If the protein is one having the activity of hGH, the two amino groups for polymer attachment are preferably selected from the group consisting of the α-amino group (N-terminus) and e-amino groups of lysine residues of hGH. It has been shown that among the potential PEG attachment sites lysine residues at positions 140, 145, 38 and 70 of the mature protein sequence according to SEQ ID No. 4 are most preferred, because they are accessible to solvent, whereas the lysine residues at positions 172, 41, 158, 168 and 115 of the mature protein are less preferred, because they are involved in receptor binding (Clark et al. (1996) J. Biol. Chem. 271(36): 21969-21977). However, the present invention also relates to the conjugation of polymer groups such as PEG to the ε-amino group of lysine 41, 115, 158, 168 and/or lysine 172 of the mature protein sequence.

Further, it is preferred that the molecular weight of a polyethylene glycol moiety attached to a amino group is from 2 to 100 kDa, preferably from 5 to 60 kDa, more preferably from 10 to 30 kDa and most preferably from 12 to 20 kDa. In a further preferred embodiment of the present invention, the number n of ethylene oxide residues in a polyethylene glycol moiety is from about 40 to about 2270, more preferably from about 110 to about 1370 and most preferably from about 225 to about 680.

In a further aspect of the present invention, pharmaceutical compositions of the above-described polymer protein conjugates are provided. Such pharmaceutical compositions may be suitable for administration by injection or for oral, pulmonary, nasal or other forms of administration. In general, the present invention provides pharmaceutical compositions comprising effective amounts of di-polymer-protein conjugates of the present invention together with pharmaceutically acceptable diluents, preservatives, solubilizers, emulsifiers, adjuvants and/or carriers. Such compositions include diluents of various buffer content, such as Tris-HCl, acetate, phosphate, pH and ionic strength; additives such as detergents and solubilizing agents such as Tween 80, antioxidants such as ascorbic acid and sodium metabisulfite, preservatives such as benzyl alcohol and bulking substances such as lactose or mannitol; incorporation of the material into particulate preparations of polymeric compounds such as polylactic acid, polyglycolic acid, etc. or into liposomes. Such compositions may influence the physical state, stability, rate of *in vivo* release and rate of *in vivo* clearance of the polymer-protein conjugates according to the present invention.

As already mentioned above, a pharmaceutical preparation according to the present invention comprises at least one di-polymer-protein conjugate according to the present invention. It may be advantageous to prepare a mixture of two di-polymer-protein conjugates in order to select the proportion of the two di-polymer-protein conjugates in the mixture. Furthermore, if desired, one may prepare a mixture of various proteins with various numbers of polymer moieties attached and combine this mixture with a di-polymer-protein conjugate according to the present invention which yields a mixture with a pre-determined proportion of di-polymer-protein conjugate according to the present invention.

In another embodiment of the present invention, a defined di-pegylated protein conjugate preparation may comprise at least two isoforms, e.g. an isoform with polyethylene glycol moieties at the N-terminus and a first e-amino group of lysine such as lysine 52 of erythropoietin and an isoform with polyethylene glycol moieties at the N-terminus and a second e-amino group such as lysine 116 of erythropoietin in any defined mixed stoichiometric ratio, such as from 0.1 to 10, or from 0.5 to 2 or from 0.75 to 1.33 or about 1.

Furthermore, di-PEG protein isoforms having attached PEG molecules with different molecular weights, i.e. 12 and 20 kDa, may show a biological activity which is comparable to the biological activity of mono-pegylated protein. According to a further aspect, the present invention provides a mixed pharmaceutical preparation of PEG protein conjugates comprising at least one di-pegylated protein conjugate as described-above mixed with multi-pegylated protein conjugates wherein the proportion of said di-pegylated protein conjugate is pre-determined. In the context of the present invention, the term multi-pegylated protein conjugates means protein conjugates having more than two PEG moieties per protein molecule. The proportion of said at least one di-pegylated protein conjugate within the mixed pharmaceutical preparation ranges from 1% to 99%, preferably from 50% to 99%, more preferably from 60, 70, 80 or 90 % to 99% and most preferably from 92, 94, 96, 98 to 99%.

According to a further aspect of the present invention, a process for the preparation of a polymer-protein conjugate such as a polyethylene glycol-protein conjugate is provided which, under the conditions of a reductive alkylation, predominantly yields di-polymer-protein conjugates such as di-pegylated protein. Preferably, this reaction of a polymer reagent such as a PEG reagent with protein to di-polymer-protein conjugates is achieved by selecting reaction conditions which result in an enriched pool of di-polymer-protein conjugates, such as a suitable reaction time, a suitable protein concentration, a suitable molar ratio of polymer to protein and/or a suitable reaction temperature. It is particularly preferred to provide a process for the predominant preparation of di-pegylated PEG-protein conjugates.

In the context of the present invention, a selective or predominant or preferred reaction of polymer with protein to di-polymer-protein conjugates or a selective or predominant or preferred yield of di-polymer-protein conjugates or selectively or predominantly or preferably preparing polymer-protein conjugates having two nitrogen atoms of amino groups of the protein each conjugated with one polymer unit means that at least 20%, preferably at least 25%, more preferably at least 30% and most preferably at least 40% or at least 45% or even at least 50% or at least 60% of the products of the reaction mixture resulting from a process of the present invention are di-polymer-protein conjugates according to the present invention. In the context of the present invention, the percentage of di-polymer-protein conjugates in the reaction mixture is the amount of at least one di-polymer-protein conjugate in relation to the amount of all identified species or products in the reaction mixture determined by cation exchange chromatography using a HPLC system and mass analysis.

Further, in the context of the present invention, a selective or predominant or preferred conversion of PEG with protein to di-pegylated PEG-protein conjugates or a selective or predominant or preferred yield of di-pegylated PEG-protein conjugates or selectively or predominantly or preferably preparing polyethylene glycol-protein conjugates having two nitrogen atoms of the amino groups of the protein each conjugated with one polyethylene glycol unit means that at least 20%, preferably at least 25%, more preferably at least 30% and most preferably at least 40% or at least 45% or even at least 50% or at least 60% of the products of the reaction mixture resulting from a process of the present invention are dipegylated PEG-protein conjugates according to the present invention. In the context of the present invention, the percentage of di-pegylated PEG-protein conjugates in the reaction mixture is the amount of at least one di-pegylated PEG-protein conjugate in relation to the amount of all identified species or products in the reaction mixture determined by cation exchange chromatography using a HPLC system and mass analysis (see also Table 1).

Preferably, the reaction time of each of the two process cycles according to the present invention is from about 6 h to about 48 h, more preferably from 12 h to 32 h. Other preferred reaction times are about 7 h, about 8 h, about 9 h, about 10 h, about 11 h, about 13 h, about 14 h, about 15 h, about 16 h, about 17h, about 18 h, about 19 h, about 20 h, about 21 h, about 22 h, about 23 h, about 24 h, about 25 h, about 26 h, about 27 h, about 29 h, about, 30 h, about 31 h and about 33h, about 34 h or about 35 h. However, the person skilled in the art is aware of the fact that the reaction time is interrelated with other process parameters such as the reaction temperature. In this respect, Chamov et al. (Bioconjugate Chemistry (1994) 5(2):133-140) have demonstrated that increasing the reaction temperature decreased the reaction time at least by a factor of 2. Moreover, it has been found for non-glycosylated proteins that the reaction time also depends on the molar ratio of the polymer to the protein. For example, a doubling of the polymer amount for a given protein concentration may decrease the reaction time for the formation of di-polymer-protein conjugates by a factor of 2.

It is preferred that the reaction is performed at a temperature from 2°C to 50°C, more preferably at a temperature from 2°C to 8°C and most preferably about 4°C. As already mentioned above, the selection of a specific temperature may affect the reaction time, which is to be chosen such that polymer-protein conjugates with two nitrogen atoms of amino groups of the protein being conjugated with a polymer unit via an amine linkage are preferably prepared. In particular, the reaction time, which is preferably from about 6 h to about 48 h, may also be lower than 6 h, e.g. 1 h, 2 h, 4 h, or 5 h, when the reaction temperature is increased. For example, the reaction time is at least 6 h, preferably from 6 h to 12 h or from 8 h to 10 h when the reaction is performed at a temperature from 15°C to 25°C, preferably from 18°C to 24°C and most preferably 22°C and at least 18 h when the reaction is performed at 5°C.

Further, it is preferred that the reaction is performed at a protein concentration from 0.5 to 100 mg/ml, more preferably from 1 to 10 mg/ml and most preferably from 3 to 7 mg/ml.

According to a further preferred embodiment of the present invention, the reaction is performed at a protein-to-polymer molar ratio of from 1:1 to 1:400, and preferably from 1:5 to 1:50 and most preferably from 1:15 to 1:30. Within the context of the present invention, a protein-to-polymer molar ratio is meant to be the ratio between moles of protein and moles of polymer at a given protein concentration in the reaction sample.

Based on the protein to polymer molar ratio the stoichiometric ratio of amino groups within the protein to polymer molecules can be deduced. Within the context of the present invention, a stoichiometric ratio of amino groups to polymer molecules is meant to be the number of α- and ε-amino groups within the protein to the number of protein molecules.

It has been found that for non- glycosylated proteins an at least 1.25 fold excess, e.g. in the case of interferon α 2a, of the number of polymer molecules to the number of amino groups is necessary to predominantly produce proteins conjugated with two polymer groups, whereas for glycosylated proteins predominantly di-polymer protein conjugates are even produced when the number of amino groups is in excess to the number of polymer molecules.

It is therefore preferred that, for non-glycosylated proteins, the stoichiometric ratio of the number of amino groups to the number of polymer molecules is in a range from 1:1 to 1:80, preferably 1:1.25 :1:80, more preferably from 1:1.25 to 1:50, even more preferably from 1:1.25 to 1:30 and most preferably from 1:2 to 1:10.

It is further preferred that, for glycosylated proteins, the stoichiometric ratio of the number of amino groups within the protein to the number of polymer molecules is in a range from 10:1 to 1:50, preferably from 1:0.3 to 1:50, more preferably from 2:1 to 1:25 and even more preferably from 1:0.8 to 1:10. Most preferably, the stoichiometric ratio is 10:1, 5:1, 1:0.3, 1:0.4, 2:1, 1:0.6, 1:0.7, 1:0.8, 1:0.9 or 1:1. Other preferred stoichiometric ratios are 1:2 to 1:10 or 1:2 to 1:5.

In a further preferred embodiment of the invention the reaction time is from about 6 h to about 48 h, more preferably from 12 h to 32 h. Other preferred reaction times are about 7 h, about 8 h, about 9 h, about 10 h, about 11 h, about 13 h, about 14 h, about 15 h, about 16 h, about 17h, about 18 h, about 19 h, about 20 h, about 21 h, about 22 h, about 23 h, about 24 h, about 25 h, about 26 h, about 27 h, about 29 h, about, 30 h, about 31 h and about 33h, about 34 h or about 35 h and the protein-to-polymer molar ratio is from 1:1 to 1:400, and preferably from 1:5 to 1:50 and most preferably from 1:15 to 1:30.

In a further preferred embodiment of the invention the reaction time is from about 6 h to about 48 h, more preferably from 12 h to 32 h. Other preferred reaction times are about 7 h, about 8 h, about 9 h, about 10 h, about 11 h, about 13 h, about 14 h, about 15 h, about 16 h, about 17h, about 18 h, about 19 h, about 20 h, about 21 h, about 22 h, about 23 h, about 24 h, about 25 h, about 26 h, about 27 h, about 29 h, about, 30 h, about 31 h and about 33h, about 34 h or about 35 h and the stoichiometric ratio of the number of amino groups to the number of polymer molecules is in a range from 1:1 to 1:80, preferably 1:1.25 :1:80, more preferably from 1:1.25 to 1:50, even more preferably from 1:1.25 to 1:30 and most preferably from 1:2 to 1:10 for non-glycosylated proteins.

In a further preferred embodiment of the invention the reaction time is from about 6 h to about 48 h, more preferably from 12 h to 32 h. Other preferred reaction times are about 7 h, about 8 h, about 9 h, about 10 h, about 11 h, about 13 h, about 14 h, about 15 h, about 16 h, about 17h, about 18 h, about 19 h, about 20 h, about 21 h, about 22 h, about 23 h, about 24 h, about 25 h, about 26 h, about 27 h, about 29 h, about, 30 h, about 31 h and about 33h, about 34 h or about 35 h and the stoichiometric ratio of the number of amino groups within the protein to the number of polymer mo lecules is in a range from 10:1 1 to 1:50, preferably from 1:0.3 to 1:50, more preferably from 2:1 to 1:25 and even more preferably from 1:0.8 to 1:10 and most preferably, the stoichiometric ratio is 10:1, 5:1, 1:0.3, 1:0.4, 2:1, 1:0.6, 1:0.7, 1:0.8, 1:0.9 or 1:1 for glycosylated proteins.

In a further preferred embodiment of the invention the reaction time is from about 6 h to about 48 h, more preferably from 12 h to 32 h. Other preferred reaction times are about 7 h, about 8 h, about 9 h, about 10 h, about 11 h, about 13 h, about 14 h, about 15 h, about 16 h, about 17h, about 18 h, about 19 h, about 20 h, about 21 h, about 22 h, about 23 h, about 24 h, about 25 h, about 26 h, about 27 h, about 29 h, about, 30 h, about 31 h and about 33h, about 34 h or about 35 h and the reaction is performed at a temperature from 2°C to 50°C, more preferably at a temperature from 2°C to 8°C and most preferably about 4°C and the protein-to-polymer molar ratio is from 1:1 to 1:400, and preferably from 1:5 to 1:50 and most preferably from 1:15 to 1:30.

In a further preferred embodiment of the invention the reaction time is from about 6 h to about 48 h, more preferably from 12 h to 32 h. Other preferred reaction times are about 7 h, about 8 h, about 9 h, about 10 h, about 11 h, about 13 h, about 14 h, about 15 h, about 16 h, about 17h, about 18 h, about 19 h, about 20 h, about 21 h, about 22 h, about 23 h, about 24 h, about 25 h, about 26 h, about 27 h, about 29 h, about, 30 h, about 31 h and about 33h, about 34 h or about 35 h and the reaction is performed at a temperature from 2°C to 50°C, more preferably at a temperature from 2°C to 8°C and most preferably about 4°C and the stoichiometric ratio of the number of amino groups to the number of polymer molecules is in range from 1:1 to 1:80, preferably 1:1.25 :1:80, more preferably from 1:1.25 to 1:50, even more preferably from 1:1.25 to 1:30 and most preferably from 1:2 to 1:10 for non-glycosylated proteins.

In a further preferred embodiment of the invention the reaction time is from about 6 h to about 48 h, more preferably from 12 h to 32 h. Other preferred reaction times are about 7 h, about 8 h, about 9 h, about 10 h, about 11 h, about 13 h, about 14 h, about 15 h, about 16 h, about 17h, about 18 h, about 19 h, about 20 h, about 21 h, about 22 h, about 23 h, about 24 h, about 25 h, about 26 h, about 27 h, about 29 h, about, 30 h, about 31 h and about 33h, about 34 h or about 35 h and the reaction is performed at a temperature from 2°C to 50°C, more preferably at a temperature from 2°C to 8°C and most preferably about 4°C and the stoichiometric ratio of the number of amino groups within the protein to the number of polymer molecules is in a range from 10:1 to 1:50, preferably from 1:0.3 to 1:50, more preferably from 2:1 to 1:25 and even more preferably from 1:0.8 to 1:10 and most preferably, the stoichiometric ratio is 10:1, 5:1, 1:0.3, 1:0.4, 2:1, 1:0.6, 1:0.7, 1:0.8, 1:0.9 or 1:1 for glycosylated proteins.

If the protein is interferon α 2a, the reaction time is preferably from 12 h to 24 h and the reaction temperature is preferably about 4°C and the stoichiometric ratio of the number of amino groups within the protein to the number of polymer molecules is preferably in the range from 1:1 to 1:10, most preferably it is 1:1.25.

If the protein is erythropoietin, the reaction time is preferably from 12 h to 24 h and the reaction temperature is preferably about 4°C and the stoichiometric ratio of the number of amino groups within the protein to the number of polymer molecules is preferably in the range from 1:0.5 to 1:8, most preferably it is 1:0.625.

If the protein is hGH, the reaction time is preferably from 12 h to 24 h and the reaction temperature is preferably about 4°C and the stoichiometric ratio of the number of amino groups within the protein to the number of polymer molecules is preferably in the range from 1:1 to 1:8, most preferably it is 1:1.6.

The reducing agent used in the reductive alkylation is preferably selected from, but not limited to, NaCNBH₄ or NaBH₄.

Further, it is preferred that the reaction be performed in the vicinity of neutrality, e.g. at a pH from 6 to 8, more preferably at a pH from 6.9 to 7.8, and most preferably at a pH from 7.2 to 7.5, especially if the protein is a protein having the biological activity of G-CSF, erythropoietin, hGH or interferon. For other proteins, it may be preferable to select a pH from 4.5 to 7.5 of 5.0 to 6.5 or about 5.5.

In order to maintain the pH in the preferred range, the reaction may be performed in the presence of a buffer that, e.g., may be selected from a phosphate, acetate, HEPES, MES, and/or bicine buffers.

The PEG reagent used in the process according to the present invention is usually a reagent having the formula

[R-L¹-(CH₂-CH₂-O)ₙ]_{y}-L²-(CH₂-)ₘ₋₁-CHO

wherein R is H, a lower alkyl, aryl or any suitable protecting group; n is an integer representing the number of ethylene oxide residues in a polyethylene glycol moiety; m is an integer representing the number of methylene groups; L¹ is O, N, S and/or a branched or non-branched linker moiety which can be absent or present; L² is a branched or non-branched linker moiety which can be absent or present; and y is a integer with the proviso that y is 1 in the absence of L² and y is at least 1 in the presence of L².

Preferably, R, m, n, L¹, L² and y are defined as described above for the polymer-protein conjugates according to the present invention.

Further, it is preferred that the polymer reagent is a PEG aldehyde, more preferably a PEG acetaldehyde, most preferably methoxy polyethylene glycol acetaldehyde (mPEG acetaldehyde).

The polyethylene glycol having a single aldehyde group, which is to be reacted with the protein, may be stored in the form of the corresponding acetal. The PEG acetal precursor may be activated to the PEG aldehyde reagent at any time before the conversion with the protein to be conjugated, under the proviso that the activated PEG aldehyde is stable until it is conjugated with the protein according to the process of the present invention. E.g., in order to obtain a polyethylene glycol reagent having a single acetaldehyde group, a corresponding polyethylene glycol diethyl acetal may be activated.

Related to this issue, a process for the long-term storage of a polyethylene glycol acetaldehyde such as methoxy polyethylene glycol acetaldehyde is provided, wherein the polyethylene glycol acetaldehyde is stored as a solid substance under an inert atmosphere such as nitrogen and at a temperature of about -20°C or less. According to this process, a polyethylene glycol acetaldehyde such as methoxy polyethylene glycol acetaldehyde may be stored for at least 1 month, preferably at least 2 months, more preferably at least 3 months and most preferably at least 4 or 6 months.

According to another preferred embodiment of the process according to the present invention, the process further comprises a separation step or purification step leading to a pool of di-polymer-protein conjugates, preferably di-pegylated polyethylene glycol-protein conjugates, wherein two defined amino groups of the protein are each conjugated with one polymer unit, also referred to as the product pool, and to a pool which contains unreacted protein, unreacted polymer reagent, di-polymer-protein conjugate isoforms other than those of the product pool and/or polymer-protein conjugates with less or more than two amino groups of the protein conjugated with a polymer unit, also referred to as the rest pool.

This separation step or purification step yielding a product pool and a rest pool is preferably performed by ion exchange chromatography.

The chemistry and the mild reaction conditions of the process according to the present invention allow recyclisation of fractions of non-desired stable reaction products for another process according to the present invention, e.g. another di-pegylation process according to the present invention. Hence, according to a further preferred embodiment, the rest pool is again subjected to the process according to the present invention, e.g. instead of using solely unpegylated protein in the conversion with PEG reagent, the rest pool is reacted with a polyethylene glycol reagent having a single aldehyde group, e.g. an acetaldehyde group, under conditions of a reductive alkylation, wherein the reaction time and/or the reaction temperature and/or the molar ratio and/or the stoichiometric ratio is chosen such that polyethylene glycol-protein conjugates having two nitrogen atoms of amino groups of the protein each conjugated with one polyethylene glycol unit are selectively prepared. The reaction time, the reaction temperature, the molar ratio and the stoichiometric ratio are selected as described above for the process of the present invention.

This preferred recyclisation step enables to significantly increase the yield of di-polymer-protein conjugate such as di-pegylated PEG-protein conjugates according to the present invention. In particular, di-pegylated PEG-protein conjugate isoforms having PEG moieties attached to the nitrogen atoms of amino groups at defined positions of the amino acid chain of the protein may be obtained.

In the context of this invention, a di-pegylated protein conjugate broadly includes any protein, which is conjugated with two polyethylene glycol units, wherein both of the polyethylene glycol units are each attached to one amino group of the protein. The di-pegylated protein conjugates of the present invention are preferably obtained by reacting a polyethylene acetaldehyde molecule with the protein under conditions of a reductive alkylation which selectively leads to a di-pegylated PEG protein conjugate having a C₂ linker between the amine group resulting from the amino group of the protein and the chain of ethylene oxide residues of the polyethylene glycol unit.

The features and advantages of this invention will become notable in more detail from the ensuing description of further preferred embodiments. In these embodiments, which include a best mode, IFN α 2a, erythropoietin and human growth hormone are exemplified as the protein and PEG is exemplified as the polymer moiety. This should, however, not be construed as a limitation of this invention to conjugates of PEG with these proteins, although such di-pegylated PEG protein conjugates are specifically preferred.

One aspect of the present invention relates to a method for preparing di-pegylated protein conjugates by attaching polyethylene glycol aldehyde moieties to the a- and e-amino groups of proteins under the conditions of a reductive alkylation.

A first step in this method can optionally be the provision of the corresponding polyethylene glycol reagent, which is to be converted with the protein. In a preferred embodiment, the polyethylene glycol reagent is a polyethylene glycol acetaldehyde, more preferred a methoxy polyethylene glycol acetaldehyde. US 5,252,714 describes that methoxy PEG acetaldehyde is unstable in aqueous solution due to aldol decompositions and oxidation. Hence, in one embodiment of the present invention, the process for providing the polyethylene glycol reagent comprises the activation of methoxy PEG di-ethyl acetal which is a stable storage form of the methoxy PEG acetaldehyde, into methoxy PEG acetaldehyde.

However, it was surprisingly found that, when stored as a solid substance, under nitrogen and at low temperatures, e.g. of at -20°C or less, the activity of the PEG acetaldehyde could be conserved for at least 4 months, preferably for at least 6 months and most preferably for at least one year, thereby allowing reproducible di-pegylation during that period. In the context of the present invention, reproducible di-pegylation means that the yield of di-pegylated protein and the reaction kinetics are comparable. Hence, in an alternative embodiment of the present invention it is not necessary that the PEG acetaldehyde is provided immediately before the reaction with the protein. Rather, the PEG di-ethyl acetal precursor may be activated into PEG acetaldehyde, preferably methoxy PEG acetaldehyde, at some hours, e.g. 12 hours or 24 hours or preferably some weeks, e.g. one or two weeks or even some months, e.g. two, three or four months or more before the reaction with the protein, preferably G-CSF.

Further, parameters have been identified which influence the yield and/or the reaction kinetics of the di-pegylation process including the recycling step according to the present invention. These parameters include protein concentration, protein-to-PEG molar ratio, stoichiometric ratio of the number of amino groups within the protein to the number of polymer molecules depending on whether the protein is glycosylated or not, pH, the reducing agent used in the reductive alkylation, temperature, incubation time, co-solvent, stirring speed and method, ionic strength of the buffer.

WO 96/11953 teaches that a sufficiently acidic pH selectively leads to mono-pegylation of α-amino groups in view of the pKa difference between a- and e- amino groups. WO 03/049699 describes that pegylation at a pH in the range from 5.5 to 7.5 only occurred at a-amino groups, which results in mono-pegylated protein. Chamow et al. (Bioconjugate Chemistry (1994) 5(2): 133-140) describe that at pH 7.5 predominantly the a-amino group of CD4-IgG is pegylated, because it is unprotonated, while the e-amino groups remain unpegylated due to their protonation. This results in essentially monopegylated protein molecules.

However, it has now been found that parameters could be selected for the process according the present invention which yield preferably di-pegylated protein by taking advantage of the different reactivity detected between different e- amino groups of the protein. For dipegylated IFN α 2a, erythropoietin and hGH, the pegylation reaction according to the present invention is preferably performed in the vicinity of neutrality, e.g. at pH 6.5 to 7.5. For other di-polymer protein conjugates pegylation reaction according to the present invention, it may be preferable to adjust a more acidic or more basic pH, e.g. at pH 3, 4, 5, 5, 8, 9, or 10.

In particular, it was found that one crucial parameter for the degree of pegylation of proteins such as erythropoietin is the reaction time. In a preferred embodiment, the preferred reaction time is chosen such that at least 20% of the reaction mixture comprises di-pegylated PEG protein conjugates, i.e. polyethylene glycol-protein conjugates, having two amino groups of the protein each conjugated with one polyethylene glycol unit. In other preferred embodiments, the reaction time for the di-pegylation reaction is chosen such that at least 25, at least 30, at least 40 or at least 50% of the reaction mixture comprises di-pegylated protein such as di-pegylated erythropoietin.

In particular, it was found that for a defined set of parameters, such as protein concentration, protein-to-PEG molar ratio, stoichiometric ratio of the number of amino groups within the protein and the number of polymer molecules, pH and temperature, a specific time frame for the incubation of the polyethylene glycol reagent, preferably polyethylene glycol acetaldehyde, more preferably methoxy polyethylene glycol (mPEG) acetaldehyde, with the protein, preferably IFN α 2a, erythropoietin and hGH, exists which favours the formation of di-pegylated PEG protein conjugates. As shown in Figure 3 for the comparative example of G-CSF, the yield of each mPEG-G-CSF conjugate, i.e. mono-, di-, tri- and tetra-pegylated species, varies depending on the incubation time. Within the initial period of incubation, the yield of mono-pegylated species reaches a maximum and starts to decline (data not shown) while di- and tri-PEG G-CSF conjugates are increasingly formed. Next, the di-PEG G-CSF conjugate yield reaches and maintains its maximum for several hours before starting to decrease, while the tri-PEG G-CSF conjugate is increasingly formed. Within the investigated incubation time, tri- and higher pegylated conjugates do not reach their yield maxima.

Hence, it has been found that the selection of the defined time frame for the reaction time of a process according to the present invention is essential for the production of specific isoforms or species of pegylated proteins such as erythropoietin, interferon or hGH at high yield.

Another critical parameter for the yield of dipegylated proteins is the molar ratio of protein to PEG and the stoichiometric ratio of the number of amino groups within the protein and the number of polymer molecules which differs for glycosylated and non-glycosylated proteins.

Usually, the process according to the present invention leads to a product mixture of differently pegylated forms, which is highly enriched in di-pegylated protein conjugate, i.e. preferably at least 20%, or more preferably at least 30% of the reaction mixture comprises di-pegylated protein conjugates. However, the reaction mixture also contains unreacted protein, unreacted polymer and otherwise pegylated protein conjugates. It is, therefore, preferred that the process according to the present invention further comprises a purification step in order to separate the di-pegylated species from undesired substances, e.g. by chromatography, preferably by an ion exchange chromatography step, more preferably by a cation exchange chromatography step (C-IEX). In this purification step, the di-pegylated protein conjugates may be separated from unreacted protein, unreacted PEG reagent and/or polyethylene glycol conjugates which have more or less than two polyethylene glycol units per protein. Further, di-pegylated protein conjugate isoforms may be separated which differ in their PEG attachment sites form the desired di-pegylated PEG protein conjugate products. The pool comprising the di-pegylated protein conjugate products is also designated as the product pool, whereas the pool comprising the undesired compounds is also designated as the rest pool, or product-depleted eluate.

In a further preferred embodiment of the present invention, the overall yield of di-pegylated protein conjugate could be further increased by recyclisation the product-depleted eluate resulting from a purification step such as the C-IEX step, in another pegylation process according to the present invention. In this embodiment, the eluate of the chromatography column, e.g. a C-IEX column, is usually subjected to ultrafiltration and/or diafiltration for buffer exchange and concentration and then converted with the polyethylene glycol aldehyde reagent, preferably an acetaldehyde such as methoxy polyethylene glycol acetaldehyde.

Further preferred embodiments of the pegylation process according to the present invention will be described in more detail below.

### Activation of polyethylene glycol acetal

In the process according to the present invention, a polyethylene glycol aldehyde having the formula

(R-O-(CH₂-CH₂-O)ₙ)_{y}-CH₂-CHO

is preferably used for the conjugation of proteins, wherein R is H or lower alkyl such as methyl; n is an integer representing the number of ethylene oxide residues in a polyethylene glycol moiety, preferably from 225 to 680; m is an integer representing the number of methylene groups; L¹ is absent; L² is absent; and y is 1. The skilled person is well aware of protocols and conditions for producing these compounds and other polymer reagents according to the present invention (see, e.g. EP 0 154 316; US 5,990,237; Chamov et al., Bioconjugate Chem., 1994, 5:133-140).

In one embodiment, the process of the present invention is started by converting a precursor compound of the polyethylene glycol reagent, such as methoxy polyethylene glycol acetal, to the activated polyethylene glycol acetaldehyde such as methoxy polyethylene glycol aldehyde. This activation is usually performed in an aqueous solution, e.g. for up to 3h, at an acidic pH, e.g. pH 2, which may be achieved by adding any organic and/or inorganic acid.

Usually, the conversion from polyethylene glycol acetal to polyethylene glycol aldehyde is tracked by analysing the components by ¹H-NMR spectroscopy.

### Pegylation reaction

As the protein converted in the pegylation reaction, any natural or recombinant protein or protein variant or protein mutant as obtained from any prokaryotic or eukaryotic cell may be used. Human proteins are preferred. In particular, any protein having the activity of the wild-type form, including variants, muteins, structurally or functionally equivalent protein as well as synthetic protein- like forms may be used. However, the use of a protein having the amino acid sequence of the wild-type protein is preferred.

Preferred proteins for the present invention are proteins of the interferon family, preferably IFN α 2a, erythropoietin and human growth hormone, which preferably have the amino acid sequence of SEQ ID No. 2, SEQ ID No. 3 and SEQ ID No. 4, respectively, and analogues thereof.

Further, the pegylation reaction according to the present invention is commonly performed in a suitable incubator, containing the protein such as IFN α 2a, erythropoietin or human growth hormone, a physiological buffer system, and activated polyethylene glycol aldehyde such as methoxy polyethylene glycol acetaldehyde, and a reducing agent. Further, it is preferred that the reaction mixture is gently stirred at low temperature. The reaction may be stopped by acidification of the reaction mixture.

### - Reaction buffer

Typical reaction buffers for the pegylation reaction according to the present invention include phosphate, citrate/phosphate, cacodylate, carbonate, HEPES, MES, BES, MOPS, bicine and/or other suitable buffers.

The concentration at which the buffers are used may depend on the amount of protein used. Typical concentrations range from 50 to 200 mM buffer such as phosphate buffer. However, the different ionic strengths do not affect the di-pegylated product yield.

### - Protein concentration

The concentration of protein used for the pegylation process according to the present invention depends on the physicochemical properties of the protein, such as solubility and aggregation, as well as on process economics. The higher the protein concentration, the lower the given molar ratio of protein to polyethylene glycol reagent to yield comparable product yields.

For the pegylation of proteins according to the present invention, the concentration of protein in the reaction mixture is preferably in the range from 1 to 20 mg/ml, more preferably from about 2 or 2.4 to about 5.7 or 6 mg/ml and most preferred at about 3.2 mg/ml.

### - Molar ratio of polyethylene glycol aldehyde to protein

It was found that the concentration of polyethylene glycol aldehyde moiety does not affect the final di-pegylated protein yield. However, it was found that the higher the polyethylene glycol concentration for a defined protein concentration is, the faster the reaction reaches the endpoint.

Hence, di-pegylation of proteins according to the present invention may be performed in a wide range of protein to polyethylene glycol reagent molar ratios. Preferably, molar ratios of polyethylene glycol aldehyde, such as methoxy polyethylene glycol acetaldehyde to protein of about 5:1 to 400:1 are preferred. More preferably, the molar ratio is 10:1 to 50:1, most preferably 15:1 to 30:1.

### - Stoichiometric ratio of polyethylene glycol to the amino groups within the protein

It has been found that for non- glycosylated proteins, a stoichiometric ratio of the number of amino groups within the protein to the number of polymer molecules in a range from 1:1.25 to 1:50, preferably from 1:1.25 to 1:30 and more preferably from 1:2 to 1:10 is preferred to produce proteins with two polymer groups.

For glycosylated proteins predominantly di-polymer protein conjugates are produced even when the number of amino groups to be accessible for PEG attachment is in excess to the number of polymer molecules at a given protein concentration.

Therefore, the stoichiometric ratio of the number of amino groups within the protein to the number of polymer molecules ranges for glycosylated proteins from 10:1 1 to 1:50, preferably from 1:0.3 to 1:50, more preferably from 2:1 to 1:25 and even more preferably from 1:0.8 to 1:10 and most preferably, the stoichiometric ratio is 10:1, 5:1, 1:0.3, 1:0.4, 2:1, 1:0.6, 1:0.7, 1:0.8, 1:0.9 or 1:1.

### - Molecular weight of polyethylene glycol aldehyde reagent

Di-pegylation of proteins according to the present invention may be performed in a wide range of polyethylene glycol reagent molecular weights, i.e. from 2 to 100 kDa. Preferred molecular weights of the polyethylene glycol aldehyde reagents are in the range from 10 to 30 kDa, e.g. from 12 to 20 kDa. Higher molecular weights may lead to lower di-pegylation yields and reaction mixtures may then show an increase in viscosity.

The chemical structure of the polyethylene glycol aldehyde reagent is not essential and thus not limited to be linear, but may also be branched.

### - Reaction temperature

Usually, the reaction temperature for the process according to the present invention will be in a range from about 2°C to about 50°C, more preferably between 2°C and 20°C. Preferably, the process according to the present invention is performed at lower temperature to minimize non-specific modification such as degradation and/or aggregation of protein by chemical or physical processes. Hence, the most preferred reaction temperatures for the process according to the present invention are in the range from about 2°C to about 8°C, e.g. at about 4°C.

### - Reducing agent

An influence of the reducing agent on the yield of pegylated protein conjugates is reported neither in EP 0 154 316 (100-400-fold molar excess of NaCNBH₄) nor in WO 96/11953 (75-fold molar excess of NaCNBH₄). EP 0 154 316 states that the reducing agent should be stable in aqueous solution and preferably be able to reduce only the Schiff base formed in the initial process of reductive alkylation.

Preferred reducing agents may be selected from the group consisting of sodium borohydride and sodium cyanoborohydride and should be added in excess to the protein concentration used. It was found that a low molar excess of a reducing agent, such as sodium cyanoborohydride, i.e. a less than 117-fold molar excess, decelerates the formation of di-pegylated G-CSF, but reached the same maximum di-pegylated protein conjugate yield compared to high concentrations of reducing agents such as a molar excess of up to 180-fold molar excess. Above a molar excess of sodium cyanoborohydride in the range of a 120-fold molar excess, the kinetic formation of di-pegylated protein conjugate does not depend on the concentration of reducing agent used.

### - Stirring method and speed

It has been found that incubation of pegylation reaction mixture with or without orbital stirring does not affect the final di-pegylated protein conjugate yield. Furthermore, it has been found that the higher the orbital stirring speed, the lower the final di-pegylated protein conjugate yield at the same incubation time.

### - Co-solvent

Ionic liquids have been tested as co-solvent for the regio-selective pegylation, revealing that the final di-pegylated protein conjugate yield decreases with increasing concentrations of a co-solvent such as 1-butyl-4-methyl-pyridinium-tetrafluorborate.

### Purification

As already mentioned above, the reaction mixture is usually subjected to a purification step, preferably by subjecting the reaction mixture to a cation exchange chromatography, after the pegylation reaction according to the present invention is stopped, preferred by acidification.

Cation exchange chromatography may be performed with all common and/or commercially available cation exchange matrices. Typical ion exchange resins that may be employed for the purpose of the present invention comprise SP-5PW (Tosohaas Biosciences, Germany), Source S (Amersham Biosciences, Germany), Fractogel SO₃ 650 (Merck, Germany), SP Sepharose HP (Amersham Biosciences, Germany) and SP FF Sepharose (Amersham Biosciences, Germany).

Usually, the purification step is performed at a temperature from 2°C to ambient temperature, preferably at room temperature.

For dilution, equilibration, washing and elution, any buffer may be used which is typically used for ion exchange chromatography, comprising phosphate, sodium acetate, TRIS/HCl, HEPES or other suitable buffers. Typically, the buffers are chosen depending on the protein being analysed and are adjusted to pH values from 3.5 to 8.5, preferably from 4 to 6.

Preferably, the dilution, washing, and equilibration buffer contains 15 mM sodium acetate at pH 4.2. The elution buffer may be of the same composition as the equilibration buffer and may contain about 1 M NaCl.

In a preferred embodiment for the chromatography step, the reaction mixture may be diluted, e.g. 3- fold with equilibration buffer in order to reduce the conductivity to approximately 6 mS/cm. This provides conditions for the complete binding of the protein conjugate according to the present invention to the ion exchange column.

Purification by C-IEX typically includes the following steps:
● column equilibration with 1 column volume buffer A;
● sample loading;
● column washing with 0.2-1 column volume; and/or
● linear gradient elution with 1M NaCl in buffer from 0 to 35% in approximately 17 column volume.

As an example, linear flow rates of about 68 cm/h may be used for all chromatography steps except sample loading which may be done at 17 cm/h.

In a preferred embodiment of the purification step of the process according to the present invention, two pools are formed from the eluate. The first pool, also designated as product pool I, contains fractions of one or two di-pegylated isoforms of the protein, also designated as the first and/or second di-pegylated fraction or species of di-pegylated protein such as G-CSF. The second pool, also designated as rest pool, contains the other protein fractions, which may contain non-pegylated, mono-pegylated, di-pegylated isoforms, which are not identical to the first and/or the second di-pegylated isoform, and higher pegylated protein fractions. The rest pool may also contain traces of di-pegylated isoforms of the product pool but essentially does not comprise di-pegylated isoforms of the product pool. This rest pool is preferably recycled in another pegylation reaction, which is performed as described above for the process according to the present invention.

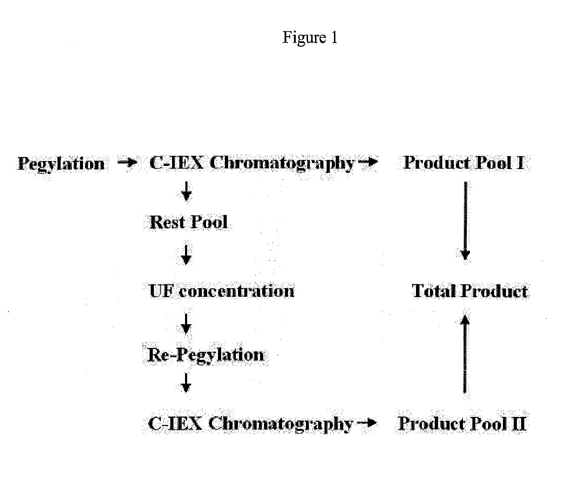

In this recycling or recyclisation or re-pegylation step, the rest pool is usually applied to ultrafiltration / diafiltration in order to concentrate the modified protein and to exchange the buffer for a buffer suited to perform a pegylation reaction according to the process of the present invention. The rest pool is then subjected to a pegylation reaction as described above for the process of the present invention, optionally followed by cation exchange purification as described above. The product pool II resulting from this re-pegylation step, optionally purified, may then be combined with the product pool I (see Figure 1).

The following examples are provided to illustrate the invention described herein and are not intended to limit it in any way.

### Examples

### A) Comparative Example: Pegylation of G-CSF with 12 kDa mPEG acetal

### 1. Preparation of the polyethylene glycol reagent

### a) Activation of mPEG acetal to mPEG aldehyde

150 mg 12 kDa mPEG acetal was dissolved in 80 mM phosphoric acid and hydrolyzed for 3h at 50°C. After cooling down on ice to approximately 2-8°C the solution was neutralized to pH 7 by drop wise addition of sodium bicarbonate solution (5%). The mixture was saturated with NaCl and three times extracted with 2 ml methylene chloride. The three collected organic phases were combined, dried by adding disodium sulphate and the volume was reduced to 1.5 ml (nitrogen stream).

mPEG acetaldehyde was precipitated by adding 30 ml ice-cold diethyl ether. The precipitate was isolated by vacuum filtration (G4 funnel), dried at room temperature under vacuum and transferred in gas-tight tubes, which were flushed with nitrogen gas before sealing them and stored at -20°C.

### b) Stability of activated mPEG aldehyde

According to the prior art, the mPEG acetal was usually activated freshly for each pegylation reaction, or was at maximum stored over one night.

To investigate the stability of activated mPEG aldehyde, which was stored at -20°C under nitrogen, stored mPEG aldehyde was subjected to the pegylation reaction (see below). The yield of di-PEG G-CSF conjugate and the kinetic of product formation served as read-outs.

### c) Analytics of polyethylene glycol reagents

To assess the quality of acetal to aldehyde conversion, three parameters were evaluated:
- Detected activity:
   ¹H-NMR spectroscopy (400 MHz spectrometer, CDCl₃ as solvent) of mPEG acetal and activated mPEG aldehyde to determine the degree of acetal to aldehyde conversion
- Mass yield activity:
   Tarring of dried activated mPEG aldehyde to determine the amount of recovered educts with respect to the mPEG acetal amount applied
- Pegylation kinetics and species yield:
   Analytical C-IEX HPLC to determine the species distribution in the reaction mixture (see pegylation reaction)

### d) Results

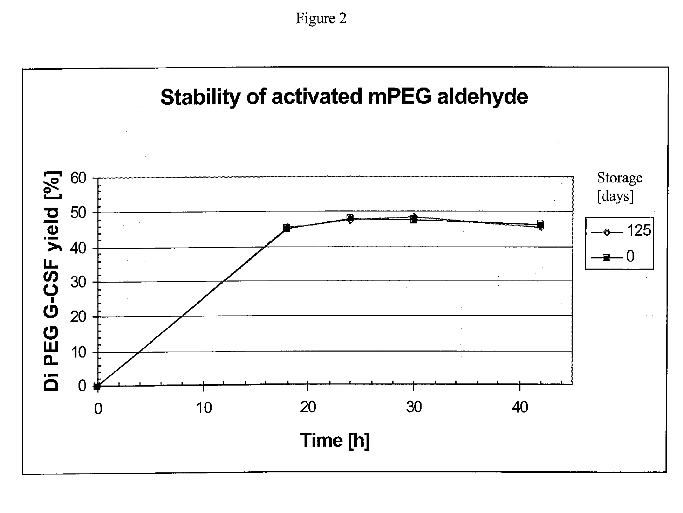

Consistency of the repeated activation of mPEG acetal to mPEG aldehyde could be documented by:
- 85 ± 7% mPEG aldehyde yield on average after activation compared to the employed amount of mPEG acetal.
- Repeated ¹H-NMR spectra of both samples displayed the PEG backbone absorption at 3.51 ppm. mPEG acetal shows the acetal triplet at 4.6 ppm (-CH₂-CH-(O-C₂H₅)₂) and the ethyl triplet absorption at 1.1 ppm (-CH₂-CH₃). After activation, the acetal triplet signal was not detectable anymore, whereas the aldehyde singlet appeared at 9.7 ppm.
- Equivalent maximum di-PEG G-CSF conjugate yields and equivalent kinetic of product formation (see below).
- Activated mPEG aldehyde appears to be stable for at least 125 days when stored light-protected, in sealed tubing, under N₂ atmosphere and at -20°C (see Figure 2).

### 2. Ultrafiltration concentration

Prior to the pegylation reaction, the diluted G-CSF sample was concentrated by ultrafiltration. Depending on the final sample concentration, G-CSF was concentrated to approximately 4-8 mg/ml using an Amicon cell (50 ml) or centricon tubes (2 ml) equipped with YM 10 membranes of 10 kDa molecular weight cut off.

Recovery yield after ultrafiltration was 89 ± 5% on average with respect to the applied protein amount.
- UV 280 nm absorption
   For G-CSF, an absorbance of 0.86 ± 0.015 at 280 nm corresponds to 1 mg/ml protein. To calculate the measured protein concentration of pegylated G-CSF conjugates, the same molar extinction coefficient was applied.

### 3. Pegylation reaction

### a) Reaction

25 mg solid mPEG aldehyde (molecular weight 12 kDa) were added to 3.2 mg/ml G-CSF in 100 mM phosphate buffer, pH 7.5, resulting in a 1:25 molar ratio of G-CSF to mPEG aldehyde. 20 mM sodium cyanoborohydride were added, and the reaction mixture was further incubated by gentle stirring at 2-8°C for several hours. The reaction was stopped by acidification with 8 N HC1.

### b) Analytics

- Analytical cation exchange high performance liquid chromatography (C-IEX HPLC)
   The species distribution (mono-, di-, tri-, tetra-) in the PEG G-CSF reaction mixture was determined by C-IEX HPLC. Therefore, the stopped reaction mixture was diluted for three times with 15 mM acetate buffer (pH 4.2) and passed through a column packed with SP-5PW (75 x 7.5 mm, 10 µm particle size, 3.3 ml bed volume, manufactured by Tosohaas Bioscience), mounted on a Dionex system or Shimadzu HPLC system.
   The column was equilibrated in 15 mM NaAc, pH 4.2. The flow rate was 0.4 ml/min and typically 200 µg of protein were injected. The column was run at room temperature; however, the samples were maintained at 5°C in the auto-injector.
   For identification of the different pegylated species, the elution peaks of the analytical C-IEX were mass analysed.
- Mass spectrometry
   By mass analysis (MALDI-TOF, matrix-assisted laser desorption/ionisation - time of flight) of the C-IEX HPLC peaks, each pegylated G-CSF conjugated could be allocated to the retention time of an elution peak.

### c) Results

The mass of the single pegylated G-CSF species determined by MALDI-TOF agreed with the preliminary mass estimation based on the amino acid sequence and PEG moiety (see Table 1).

**Table 1: Comparison of calculated and experimentally determined molecular weight of different pegylated G-CSF species.**

| **Molecular weight (estimated)** | **Molecular weight (MALDI-TOF)** | **Number of PEG molecules attached** | **Abbreviated name** |
|---|---|---|---|
| **[kDalton]** | **[kDalton]** | | |
| 18.8 | 19.0 | - | non-modified G-CSF |
| 30.1 | 31.0 | 1 | Mono-PEG G-CSF |
| 41.5 | 43.3 | 2 | Di-PEG G-CSF |
| 52.9 | 55.3 | 3 | Tri-PEG G-CSF |
| 64.3 | 67.5 | 4 | Tetra-PEG G-CSF |

With the information from the mass analysis a retention time could be allocated to the corresponding PEG G-CSF species. The analytic C-IEX allowed the separation of one mono-PEG G-CSF, three di-PEG G-CSF isoforms, four tri-PEG G-CSF isoforms, and one tetra-PEG G-CSF in dependence of the retention time.

In general, converting recombinant human G-CSF with mPEG aldehyde in a 1:25 molar ratio to PEG G-CSF conjugates yielded predominantly di-pegylated G-CSF at a reaction time from 18 h to 42 h incubation, reaching the maximum yield at about 30 h incubation time (see Figure 3a).

### d) Optimization of process parameters

### i) Influence of some process parameters

Having identified the incubation time for maximal di-PEG G-CSF production (about 30 h) the influence of the other parameters (i) concentration of phosphate buffer, (ii) pH of the phosphate buffer, (iii) concentration of the reducing agent, cyanoborohydride, (iv) protein concentration and molar ratio protein to PEG on the product yield and (v) stirring speed was investigated.

Similar reaction kinetic profiles and maximal product yields have been achieved:
- for buffer concentrations between 50 mM and 150 mM phosphate at pH 7.5,
- for the pH range from 6.9 to 7.8 with 100 mM phosphate buffer,
- for concentrations of reducing agent ≥ 20 mM; in contrast, low concentrations such as 10 mM decelerate the di-PEG G-CSF formation (maximum product yield at 42 h),
- for protein concentrations up to 5.7 mg/ml and corresponding molar ratios (A further increase of protein concentration was avoided because of potential danger of protein aggregation. An increase of PEG concentration does not improve the maximal product yield.)
- for orbital stirring speed ≤ 700 rpm.

### ii) Influence of molar ratio

Various amounts of solid mPEG aldehyde (from 15 to 38 mg) were added to different protein concentrations (2.4 mg/ml, 3.2 mg/ml, 5.7 mg/ml) resulting in molar ratios of about 1: 10, 1:15, 1:20, 1:25, 1:30, and 1:50 (protein:PEG). The reaction was performed at low temperature (5°C) as described in example 3a and the samples were analysed according to example 3b.

At a given protein concentration, e.g 2.4, 3.2, or 5.7 mg/ml, increasing amounts of PEG expressed in molar ratios reduce the total incubation time to achieve pre-dominantly diPEGylation (see figure 3b). For all protein concentrations, it is shown that a doubling of the PEG amount reduces the incubation time by a factor of at least 2 to achieve maximal di-PEG G-CSF conjugate yield comprising di- and di'-PEG G-CSF isoforms.

At molar ratios of protein to polymer of up to of 1:30, when the stoichiometric ratio of the number of mPEG aldehyde molecules to the number of amino groups within the protein is 6:1, predominantly di-PEG G-CSF conjugate could be produced after 18h incubation, but at a higher stoichiometric ratio 10:1 of the number of mPEG aldehyde molecules to the number of amino groups within the protein as for the molar ratio of 1:50 G-CSF: PEG, the reaction and the maximal di-PEG G-CSF conjugate product formation occurs in less than 18h incubation as shown for the conditions 2.4 mg/ml G-CSF, 1:50 molar ratio G-CSF:PEG.

However, the maximum synthesis yield of the Di-PEG G-CSF conjugate (40%, see figure 3c) is not influenced by the varying stoichiometric ratios of PEG molecules to the number of amino groups within the protein.

### iii) Influence of the temperature

Besides the stoichiometric ratio protein:PEG, the temperature could have an additional influence on the incubation time for producing di-PEG G-CSF product To test the influence of temperature on di-pegylated product yield (di-PEG G-CSF isoform mixture) and kinetic of product formation, two reaction conditions (5.7 mg/ml G-CSF with PEG in either 1:10 or 1:20 molar ratios) were tested at two different temperatures (5°C and 22°C) and analysed according to the invention comparing low (5°C) and high (22°C) temperature influence.

A temperature increase by approximately + 20°C accelerates the pegylation reaction by a factor of 2 confirming the results of Chamow et al. (1994).

An increase in the total amount of PEG reduces the incubation time to achieve pre-dominantly di-PEGylation (see figure 3d). Taking twice the amount of PEG (36mg instead of 18mg) reduces the incubation time for maximal product formation by a factor of 2 (42h →~ 21h) at low (5°C) as well as at high (22°C) temperature.

Surprisingly, we found that the pegylation reaction to produce predominantly di-pegylated protein conjugates can be further accelerated by a factor of at least 2 by doubling the PEG amount for a given protein concentration in the reaction which results in an at least a 2-10 fold excess of PEG molecules in relation to the available amino groups (amino and epsilon) for PEG attachment independent of the selected temperature.

In general, to favour selectively di-PEGylation of G-CSF, (i) the pH of the reaction should be in the vicinity of neutrality (to make use of the different reactivity of the epsilon amino groups of the target protein), and (ii) PEG should be added in at least 2-10 fold molar excess to the available α- and ε-amino groups in the target protein which in the case of G-CSF corresponds to molar ratios of G-CSF:PEG between approximately 1:10 and 1:50.

### e) Reproducibility of the reaction

Five single pegylation runs carried out under the conditions listed above were analyzed using cation exchange chromatography. This demonstrated the reproducibility of the G-CSF pegylation reaction according to the present invention.

The average isoform composition in the PEG G-CSF reaction mixture at 30 h incubation was determined (see Table 2).

**Table 2: Relative percentage of differently pegylated G-CSF species in five PEG G-CSF syntheses at a 30 h reaction time with a single coupling cycle.**

| | Mono-PEG G-CSF(%) | Di-PEG G-CSF(%) | Tri-PEG G-CSF(%) | Tetra-PEG G-CSF(%) |
|---|---|---|---|---|
| Mean ± STD | 24.9±2.1 | 47.8±0.3 | 24.3± 1.8 | 2.9±0.5 |

### 4. Re-pegylation and its reproducibility

To improve the process yield, a re-pegylation of the rest pool from the preparative C-IEX chromatography (see Example 5) was performed as an additional process step.

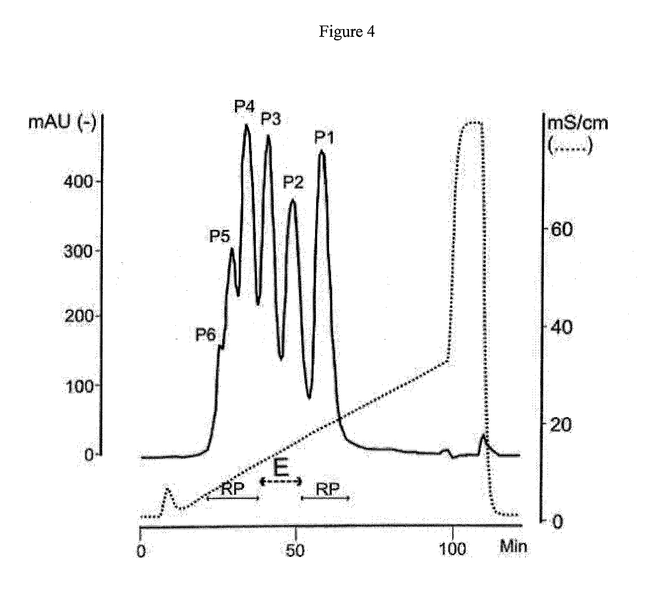

For this re-pegylation step, the rest pool of the preparative C-IEX chromatography (see Figure 4) containing all species including traces of product species according to the present invention, was collected, de-salted and concentrated by ultrafiltration/diafiltration and subjected again to the pegylation reaction (see Example 3), except that PEG modified G-CSF (3.2 mg/ml) was used.

Based on analysis of species distribution at different incubation times, it was observed that a reduction of the pegylation time to 18 h reduces the yield for the di-pegylated G-CSF conjugates by 4% on average, but increases the mono PEG G-CSF fraction within the rest pool for recyclisation by 15% on average. This was expected to increase the yield in the re-pegylation step and to lead overall to a significantly increased di-PEG G-CSF product yield of employed protein.

The average di-PEG G-CSF conjugate yield in the reaction mixture after 2 pegylation cycles of 18h, each, was determined (see Table 3).

**Table 3: Relative percentage of differently pegylated G-CSF species in six PEG G-CSF syntheses cycles comprising two coupling cycles of 18 h each.**

| | Mono-PEG G-CSF(%) | Di-PEG G-CSF(%) | Tri-PEG G-CSF(%) | Tetra-PEG G-CSF(%) |
|---|---|---|---|---|
| Mean ± STD | 18.9±3.3 | 64.1±0.7 | 33.7±3.0 | 8.0±1.7 |

### 5. Preparative C-IEX purification

### a) Chromatography

The separation of the di-pegylated G-CSF conjugates from other species after the first pegylation reaction or after re-pegylation was done by cation exchange chromatography including the following steps:
- ECO 15/200 column (Kronlab GmbH, Germany) packing with SP-5PW gel material (10.6 ml gel material, 20µm particle size, Tosohaas Bioscience GmbH, Germany);
- column equilibration with 1 column volume 15 mM NaAc buffer, pH 4.2 (buffer A);
- Protein load (6.1 mg reaction mixture);
- column washing with 0.2 CV buffer A ;
- linear gradient elution with 1M NaCl in buffer A from 0 to 35% in approximately 17 column volume; and/or
- eluent monitoring at 214 nm and 280 nm and collecting 3 ml fractions.

Linear flow rates were approximately 68 cm/h for all chromatography steps except for protein loading which was done at 17 cm/h.

### b) Analytics

- UV 280 nm absorption to determine the protein concentrations of pegylated G-CSF conjugates in each collected fraction (see above)
- Analytical cation exchange high performance liquid chromatography (C-IEX HPLC) to identify the corresponding pegylated G-CSF species and isoforms within each elution peak (see above).

### c) Results

The linear gradient elution resulted in six peaks, which are shown in Figure 4 and summarized in Table 4:

**Table 4: Allocation of elution peaks from the preparative C-IEX run with corresponding retention time and allocated PEG G-CSF species (isoforms).**

| **Peak** | **No. 6** | **No. 5** | **No. 4** | **No.3** | **No.2** | **No.1** |
|---|---|---|---|---|---|---|
| **Retention time (min)** | ~25.6 | ~29.5 | ~33.6 | ~40.5 | ~48.5 | ~57.5 |
| **PEG G-CSF species containing** | Tri"/Tri"' & traces of Tetra | Tri' & Tri" | Tri'/Di" | Di' | Di | Mono |

For di-pegylated G-CSF, three out of ten possible isoforms could be identified. Two isoforms, Di and Di' PEG G-CSF, eluted in major single peaks. The third minor isoform co-eluates with a tri-PEG G-CSF isoform.

In order to provide a proper product definition of a di-PEG G-CSF preparation, the number of isoforms was kept low defining a di-PEG G-CSF product according to the present invention which comprises two isoforms, di- (peak 2) and *di'-* (peak 3). PEG attachment sites for these isoforms have been identified to be located at the N-terminus and lysine 17 or the N-terminus and lysine 35, respectively, by using a combination of MALDI-MS/ nano-LC ESI-MS/MS and Edman sequencing (see product characterisation).

The defined di-PEG G-CSF product contained at least 90% di-PEG G-CSF species, comprising di and di' isoforms in an approximately 0.76 (di) to 1 (di') ratio and was highly pure (see Table 5)

**Table 5: Averaged di-PEG G-CSF product definition optimized for product pool I (n=6).**

| **Species-PEG G-CSF** | **Mean (%)** | **Standard-deviation (%)** |
|---|---|---|
| Mono- | 0.4 | 0.1 |
| Di- | 42.1 | 3.8 |
| Di' | 55.4 | 3.6 |
| Tri' | 2.4 | 0.7 |
| Others | 0.3 | 0.1 |

### 6. Product characterisation

The isolated di-PEG G-CSF product consists of two major (di and di') di-PEG G-CSF isoforms which represent the major active di-pegylated product species.

### a) Composition and purity

Composition and purity of the final di-PEG G-CSF product were analysed by cation exchange and size exclusion chromatography.
- C-IEX HPLC to control the composition of final di-PEG G-CSF product
   Column: Tosoh SP 5PW 10 µm 75 x 7.5 mm (3.31 ml)
   Flow rate: 0.4 ml/min
   Buffer A: 15 mM NaAc, pH 4,2
   Buffer B: 15 mM NaAc, pH 4,2; 1.000 mM NaCl
   Sample: 50 µg
   Gradient: linear, 0-45% in 17 Column volumes, 45-100% in 0.5 CV
   UV Monitor: 214 nm

### Results:

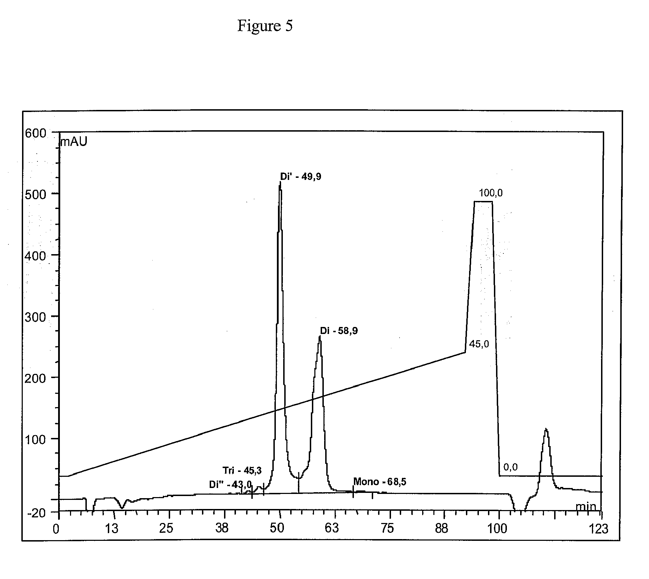

The isomer composition of the di-PEG G-CSF product was analysed by C-IEX HPLC (see Figure 5) and the composition was within the defined product specification (see Table 6).

**Table 6: Averaged Di-PEG G-CSF Product definition optimized for the total process.**

| **Species-PEG G-CSF** | **Mean (%)** | **Standard-deviation (%)** |
|---|---|---|
| Mono- | <0.5 | 0.1 |
| Di- | 43 | 4 |
| Di' | 55 | 4 |
| Tri' | 2 | 1 |
| Others | I <0.5 | 0.1 |

- SEC-HPLC analysis to control unspecific aggregation of di-PEG G-CSF species
   Column: TSK G 3000 SWXL 300 x 7,8 mm, TOSOH BIOSEP
   Buffer A: 100 mM Pi; pH 6.9
   Sample: 20 µg
   Flow rate: 0.5 ml/min
   Gradient: Isocratic
   Run time: 60 min

### Results:

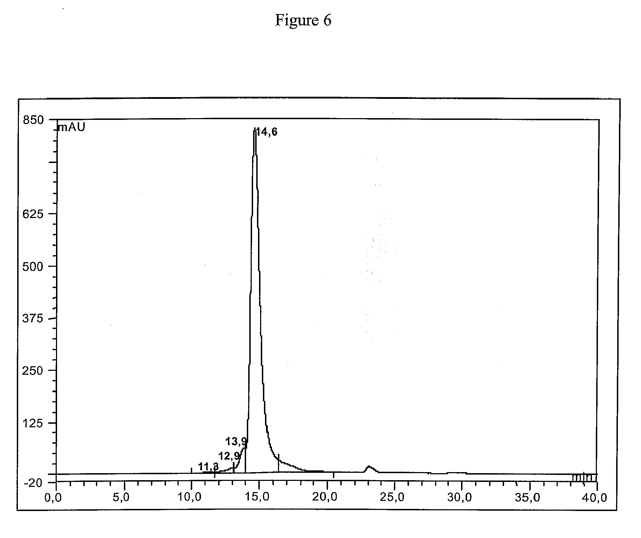

According to SEC analysis (see Figure 6), the final product consists of > 90% di-pegylated G-CSF, comprising di and di' PEG G-CSF isoforms in a defined mixed ratio. The peaks between 11 and 13 min retention time were not further analysed, but could potentially be higher molecular aggregates (~1.5 %, see Table 7).

**Table 7: PEG G-CSF isoform distribution in the final di-PEG G-CSF product analysed by SEC analysis.**

| **Species** | **Di-PEG G-CSF Product** | |
|---|---|---|
| | Retention time (min) | Rel. area (%) |
| **Mono-** | 16.8 | 4.4 |
| **Di-** | 14.6 | 90.4 |
| **Tri-** | 13.9 | 3.7 |
| **n.d.** | 12.9 | 1.2 |
| **n.d.** | 11.3 | 0.3 |

### b) Structural consistency

- Circular Dichroism (CD) measurement of di-PEG G-CSF product to show structural stability.
   In order to analyse changes in the secondary structure after protein modification, the final di-PEG G-CSF product (sample B) and native unmodified G-CSF (sample A) were analysed by circular dichroism spectroscopy. CD spectra were recorded from 180-260 nm wavelengths using a Jasco J-720 (Japan) spectropolarimeter.

### Results:

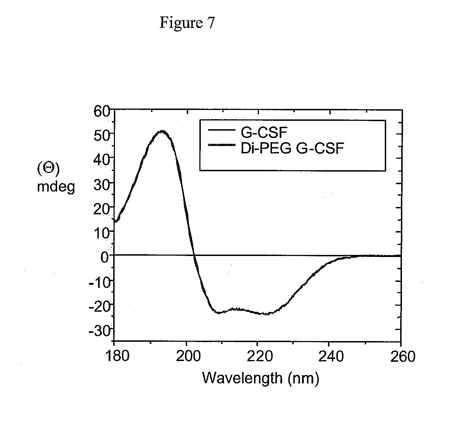

The close similarity of the se spectra (di-PEG G-CSF, unmodified G-CSF) shows that the PEG modification does not change the secondary structure of G-CSF (see Figure 7).

### c) Identification of PEG attachment sites

To identify the PEG attachment sites, the di-pegylated G-CSF isoforms (di- and di' PEG G-CSF) were analysed by peptide mapping. In order to create a reference rpHPLC chromatogram, non-pegylated G-CSF was treated identically.

The procedure comprised the steps (i) denaturation of the PEG G-CSF isoforms and capping of the free SH groups by reductive alkylation, (ii) chymotrypsin digestion of the denatured PEG G-CSF isoforms, (iii) rpHPLC peptide separation and (iv) sequence analysis by combination of electrospray tandem mass analysis and MALDI-MS/N-terminal sequencing.
(i) Carboxymethylation of SH groups
   Approximately 1 mg aliquots of di-pegylated G-CSF conjugate isoforms were dried by speed vac and reconstituted to a concentration of 1 mg in 700 µl 0.3 M Tris-HCL buffer, pH 8.3, containing 6 M guanidium hydrochlorid. Samples were carboxymethylated by adding iodoacetic acid and incubated for 1h at 37°C. The reaction was stopped by adding DTT, then the sample was dialysed against water for injection to be desalted and finally speed vac dried.
(ii) Chymotrypsin digestion
   Dried samples of di-PEG G-CSF isoforms were reconstituted to a concentration of 1mg in 1ml O.1M ammonium hydrogen carbonat buffer, pH 7.8 for digestion. The isoforms were digested with chymotrypsin (enzyme to substrate ratio per weight of 1:100) at 37°C for 3h.
(iii) rpHPLC peptide mapping
   Protein digests were injected onto a Vydac C4 column (4.6x 250 mm, 5m particle size, 300 Å pore size) and peptides were mapped by HPLC using a linear gradient of acetonitrile in 0.1% TFA (1% acetonitrile increase/min over 90 min). The resulting peptides were collected for peptide sequencing by a combination of nano-LC ESI MS/MS and N-terminal sequencing.
(iv) Nano LC - ESI/MS/MS:
   Complete digests of single isoforms as well as isolated pegylated peptides were analysed by nano LC in combination with ESI MS/MS to determine the amino acid sequence and the PEG attachment sites of the pegylated peptides.
(v) MALDI-MS / N-terminal sequencing
   Comparison of the rpHPLC chromatograms of the degradation of the di-pegylated G-CSF isoforms and the non-pegylated G-CSF revealed that two new peaks appeared. Firstly, the mass of the new peaks were determined by MALDI-MS, secondly they were N-terminally sequenced for identification.

### Results:

According to a simulated chymotrypsin digest of possible di-PEG G-CSF isoforms, three potentially pegylated peptides could be expected. Experimentally, the chymotrypsin digests of each of the two di isoforms (di and di' -PEG G-CSF) resulted in two pegylated peptides. For one pegylated peptide, peptide mass sequencing with nano-LC ESI-MS/MS identified the sequence from amino acid 1 to 13: M-T-P-L-G-P-A S-S-L-P-Q-S-F (MW 1470 m/z) having the PEG attachment to the α-amino group of the N-terminal methionine.

Identification of the other PEG attachment site per isoforms failed using this technique. Thus, selected rpHPLC fractions were mass analysed and subsequently subjected to N-terminal amino acid sequence analysis.

For the di-PEG G-CSF isoform, N-terminal sequence analysis (22 cycles) determined the sequence from amino acid 14 to 40: LLKCLEQVRKIQGDGAALQEKLCATY having the PEG attachment to the ε-amino group of lysine 17.

For the di'-PEG G-CSF isoform, N-terminal sequence analysis (22 cycles) determined the sequence from amino acid 14 to 40: LLKCLEQVRKIQGDGAALQEKLCATY having the PEG attachment to the ε-amino group of lysine 35.

Hence, the Di-PEG G-CSF isoform is di-pegylated at the N-terminus and lysine 17 and the Di'-PEG G-CSF isoform is di-pegylated at the N-terminus and lysine 35.

### d) Bioactivity

### i) Bioactivity of the di-PEG G-CSF product comprising a mixture of di- and di' PEG G-CSF conj ugate

The di-PEG G-CSF product comprising a mixture of di- and di' PEG G-CSF conjugate isoforms according to the invention was tested *in vivo,* to show the improved activity versus unmodified G-CSF (Neupogen®) and to compare its biological activity with commercially available mono-pegylated G-CSF (Neulasta®).

The *in vivo* study was an open randomized, parallel- grouped single dose biocomparability study of Neupogen® and Neulasta® with the di-PEG G-CSF product. It was carried out by subcutaneously injecting 0.1 mg/kg to male rats. Six animals per group per time point were subjected to bleeding. Serum samples were subjected to a complete blood count on the same day that the samples were collected. The average white blood cell counts and neutrophil counts were calculated.

### Results:

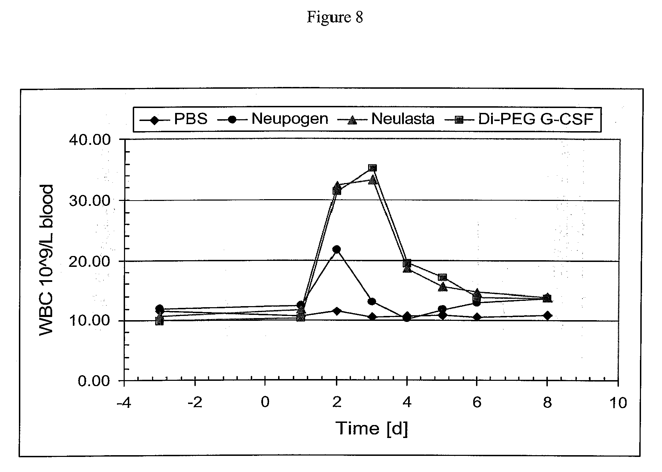

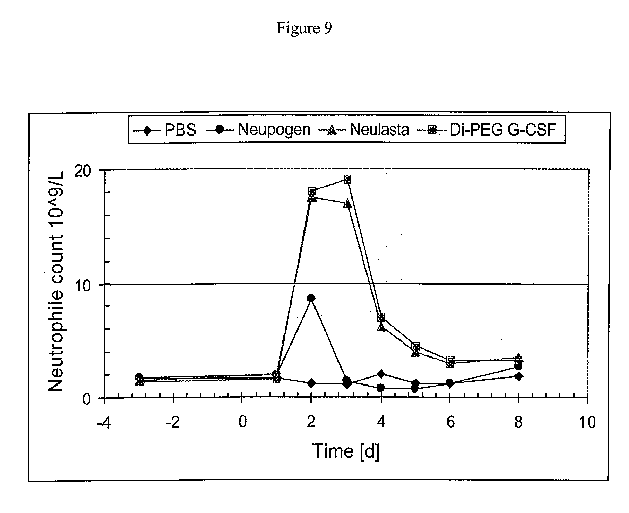

The Di-PEG G-CSF product comprising a mixture of the di- and di'-PEG G-CSF conjugate isoforms yielded higher WBC and neutrophil counts and longer sustained WBC and neutrophil response compared to unmodified G-CSF (Neupogen®) (see Figure 8 and Figure 9).

Compared to Neulasta®, the di-PEG G-CSF product according to the present invention was at least biosimilar, perhaps potentially better, because of slightly higher maximum WBC and neutrophil counts (see Figure 8 and Figure 9).

### ii) Bioactivity of the two isoforms di-PEG G-CSF and di'-PEG G-CSF

To show the contribution of each single isoform (di-PEG G-CSF, di'-PEG G-CSF) to the total bioactivity of the di-PEG G-CSF product mixture the isoforms were homogenously purified by cation exchange chromatography. Purity and composition of each isoform product (di-PEG G-CSF, di'-PEG G-CSF) were analysed by size exclusion chromatography and cation exchange HPLC.

### Results:

The defined single isoform products (di-PEG G-CSF, di'-PEG G-CSF) contained at least 95% of the respective di-pegylated G-CSF conjugate isoform (see Table 8) .

**Table 8: Isoform distribution in the defined single isoform products (di-PEG G-CSF, di'-PEG G-CSF).**

| **Species-PEG G-CSF** | **Di-PEG G-CSF isoform (%)** | **Di'-PEG G-CSF isoform (%)** |
|---|---|---|
| Di- | = 95 | 1.3 |
| Di' | 3 | = 95 |
| Others | 0.4 | 2 |

The single isoform products, di- and di'-PEG G-CSF conjugate products, and the di-PEG CSF product mixture comprising both isoforms as described in example 5 were tested *in vivo* to compare their biological activities among each other.

The *in vivo* study was an open randomized, parallel- grouped single dose biocomparability study of the mixture of both isoforms, Di-PEG G-CSF conjugate mixture as defined product according to the invention with the single isoform products, di- and di'-PEG G-CSF conjugates. The *in vivo* study was carried out by subcutaneously injecting 0.1 mg/kg of each of said products to male rats. Twelve animals per group per time point were subjected to bleeding. Serum samples were subjected to a complete blood count on the same day that the samples were collected. The average white blood cell counts and neutrophil counts were calculated.

### Results:

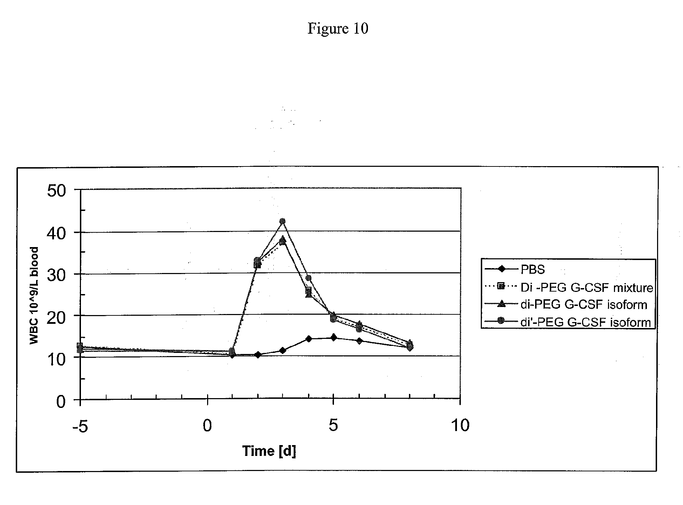

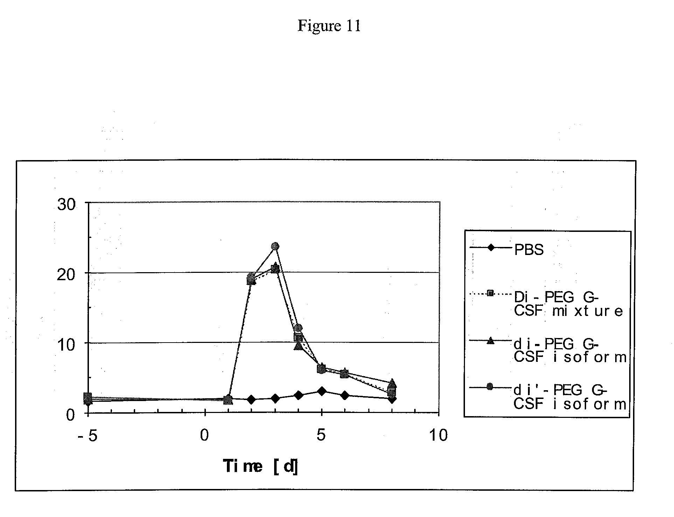

Both isoforms, di-PEG G-CSF and di'-PEG G-CSF, yielded similar high WBC and neutrophil counts and similar sustained WBC and neutrophil response compared to the Di-PEG G-CSF product mixture (see Figure 10 and Figure 11). Both isoforms are equally bioactive, thus, they uniformly contribute to the total bioactivity of the Di-PEG G-CSF product mixture. The observation is in contrast to the hypothesis that PEG modification at lysine 17 of GCSF would reduce the biological activity of this isoform compared to a PEG modification at lysine 35 of G-CSF as suggested by Aritomi et al. (Nature (1999), 401: 713-717) and Young et al. (Protein Science (1997), 6: 1228-1236).

### B) Comparative Example: G-CSF and 20 kD mPEG acetal

### 1. Pegylation reaction & Analytics

For the pegylation reaction, the educts, 20 kDa mPEG acetal and G-CSF were prepared according to examples A1a and A2. 32 mg solid mPEG aldehyde (molecular weight 20 kDa) were added to either 2.4 mg/ml G-CSF or to 3.2 mg/ml G-CSF in 100 mM phosphate buffer, pH 7.5, resulting in a 1:25 molar ratio of G-CSF (2.4 mg/ml) and in a 1:19 molar ratio of G-CSF (3.2 mg/ml) to mPEG aldehyde, respectively. The reactions were conducted and the samples were analysed by C-IEX HPLC as described in examples 3a and 3b.

### Results:

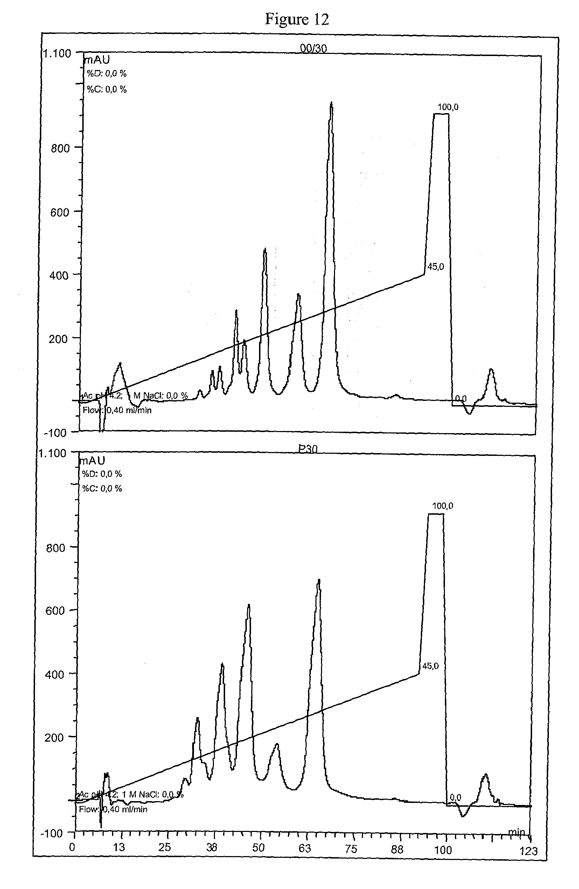

Deduced from the analytical C-IEX elution profile for mPEG 12kDa G-CSF conjugates, the corresponding mPEG 20kDa G-CSF conjugate peaks could be identified and allocated (see Figure 12). For the higher MW PEG G-CSF conjugates, the analytical C-IEX allowed the separation of one mono-PEG GCSF - (having a total MW of 39 kDa) -, two di-PEG G-CSF - (having a total MW of 59 kDa) and three tri-PEG G-CSF conjugates (having a total MW of 79 kDa).

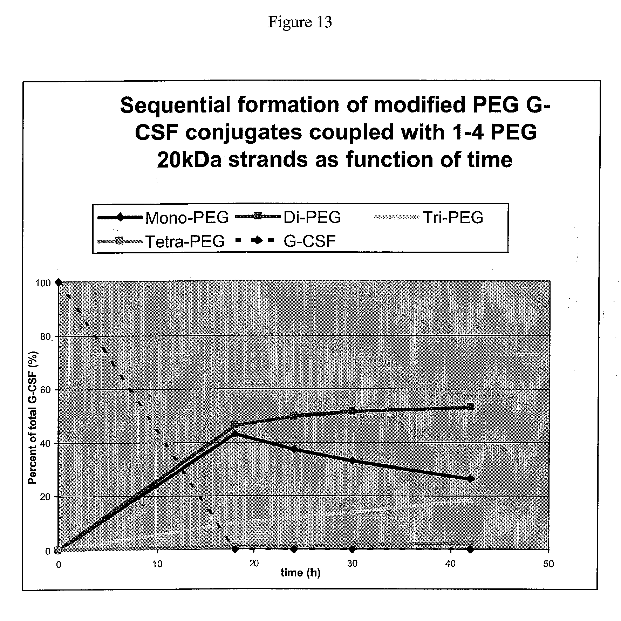

In general, converting recombinant human G-CSF with mPEG aldehyde of 20 kDa in a 1:19 or higher molar ratio to PEG-G-CSF conjugates yielded predominantly di-pegylated G-CSF conjugates with a total MW of 59 kDa after 20h incubation time at low temperature (see Figure 13).

### 2. Bioactivity of the Di'-PEG 20kDa G-CSF conjugate isoform

For testing the biological activity of the Di'-PEG 20kDa G-CSF conjugate isoform *in vivo,* the single isoform was highly purified by preparative cation exchange chromatography. The defined product contained at least 95% di'-PEG G-CSF conjugate isoform as described in example A 6, paragraph d)ii).

The *in vivo* study was an open randomized, parallel- grouped single dose biocomparability study of Neupogen® and Neulasta® with the single di'-PEG G-CSF conjugate isoform. It was carried out by subcutaneously injecting 0.1 mg/kg to male rats. Six animals per group per time point were subjected to bleeding. Serum samples were subjected to a complete blood count on the same day that the samples were collected. The average white blood cell counts and neutrophil counts were calculated.

### Results:

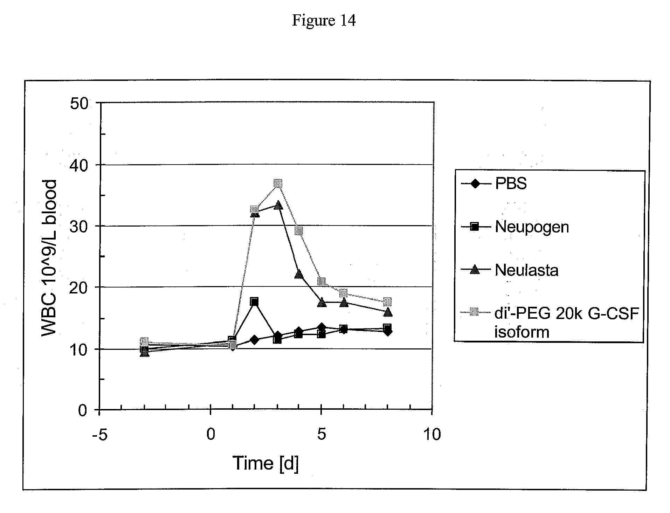

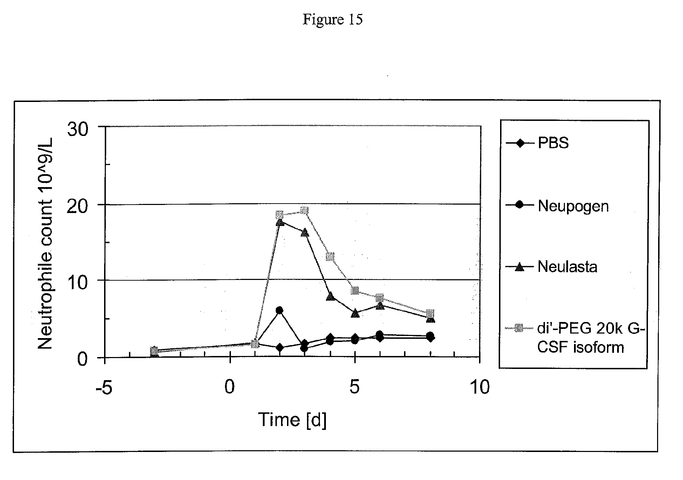

The single Di'-PEG 20kDa G-CSF conjugate isoform according to the present invention yielded i) higher WBC and neutrophil counts and ii) showed a sustained WBC and neutrophil response compared to unmodified G-CSF (Neupogen®) (see Figure 14 and Figure 15).

Compared to Neulasta®, the di'-PEG G-CSF conjugate isoform according to the present invention is bioimproved because of higher maximum WBC and neutrophil counts and its sustained WBC and neutrophil response (see Figure 14 and Figure 15)

### 3. Pharmacokinetic Investigations

The clearance mechanism of (i) the Di-PEG G-CSF product mixture (43 kDa total MW) comprising di-PEG 12kDa G-CSF and di-PEG 12kDa G-CSF conjugate isoforms according to example A6 and of (ii) the single isoform, Di'-PEG 20kDa G-CSF conjugate (59 kDa total MW) according to example B, was tested *in vivo* in comparison to commercially available mono-pegylated G-CSF (Neulasta®)

The *in vivo* studies were open randomized, parallel- grouped clearance studies of Neulasta® with either the Di-PEG G-CSF product mixture or the single di'-PEG 20kDa G-CSF conjugate isoform in sham-operated (control group) and nephrectomised rats. The studies were carried out by intravenous injection of 0.005 mg/kg of the conjugate to male rats. Four animals per group per time point were subjected to bleeding. The remaining G-CSF plasma concentration was determined in the serum samples and relevant pharmacokinetic data such as the plasma elimination half-life were calculated.

### Results:

Neulasta®, the Di-PEG 12kDa G-CSF product mixture and the single Di'-PEG 20kDa G-CSF conjugate isoform according to the present invention showed similar pharmacokinetic data (see Table 9) and cleared with a similar kinetic, which implies that all three products are predominantly cleared by Neutrophil Receptor-mediated clearance.

**Table 9: Pharmacokinetic parameters from two different PK studies with 4 rats per treatment group (SH -sham operated; NE -nephrectomised)**

| **Parameter** | **Treatment group** | **Neulasta** | **Di-PEG 12kDa G-CSF mixture** | | **Neulasta** | **Di'-PEG 20kDa G-CSF isoform** |
|---|---|---|---|---|---|---|
| **Elimination half-life (h)** | SH | 1.9 | 2.9 | | 1.9 | 3.3 |
| | NE | 2.5 | 3.4 | | 2.1 | 3.7 |

### C) Dipegylation of IFN a 2a

### 1. Pegylation reaction and Analytics

12 kDa mPEG acetal was activated for the pegylation reaction as described in example A1. Prior to the pegylation reaction, the lyophilized IFN a 2a sample was dissolved in water, dialysed against acidic acetate buffer and finally concentrated by ultrafiltration to a concentration of approximately 4-8 mg/ml depending on the reaction conditions using centricon tubes (2 ml) equipped with YM 10 membranes of 10 kDa molecular weight cut off.

For IFN a 2a, an absorbance of 1.05 at 280 nm corresponds to 1 mg/ml protein. To calculate the measured protein concentration of pegylated IFN a 2a conjugates, the same molar extinction coefficient was applied.

27 mg solid mPEG aldehyde (molecular weight 12 kDa) were added to 6 mg/ml IFN α 2a in 100 mM phosphate buffer, pH 7.5, resulting in a 1:15 molar ratio of IFN α 2a to mPEG aldehyde. 20 mM sodium cyanoborohydride were added, and the reaction mixture was further incubated by gentle stirring at 2-8°C for several hours. The reaction was stopped by acidification with 8 N HCl.

The species distribution (mono-, di-, tri-) in the PEG IFN α 2a reaction mixture was determined by C-IEX HPLC. Therefor, the stopped reaction mixture was diluted three times with 40 mM acetate buffer (pH 4.2) and passed through a column packed with SP-5PW (75 x 7.5 mm, 10 µm particle size, 3.3 ml bed volume, manufactured by Tosohaas Bioscience), mounted on a Dionex system or Shimadzu HPLC system.

The column was equilibrated in 40 mM NaAc, pH 4.2. The flow rate was 0.4 ml/min and typically 200 µg of protein were injected. The column was run at room temperature; however, the samples were maintained at 5°C in the auto-injector.

For identification of the different pegylated species, the elution peaks of the analytical C-IEX HPLC were mass analysed (MALDI-TOF) or/and analysed by SDS PAGE, which allowed the allocation of each pegylated IFN α 2a peak to the retention time of an elution peak.

Combined analysis by mass spectrometry and SDS PAGE with coomassie (protein labelling) and iodine staining (PEG labelling) agreed with the preliminary mass estimation based on the amino acid sequence and PEG moiety (see Table 10).

**Table 10: Comparison of calculated and experimentally determined molecular weight of different pegylated IFN a 2a species.**

| **Molecular weight (estimated)** | **Molecular weight (MALDI-TOF)** | **Number of PEG molecules attached** | **Abbreviated name** |
|---|---|---|---|
| **[kDalton]** | **[kDalton]** | | |
| 19.2 | n.d. | - | non-modified IFN α 2a |
| 31.2 | 31.6 | 1 | Mono-PEG IFN α 2a |
| 43.2 | 43.9 | 2 | Di-PEG IFN α 2a |
| 55.2 | 56.1 | 3 | Tri-PEG IFN α 2a |

With the information from the mass analysis / SDS PAGE a retention time could be allocated to the corresponding PEG IFN α 2a species. The analytical C-IEX allowed the separation of mono-PEG-, di-PEG- and tri-PEG-IFN α 2a isoforms in dependence of the retention time.

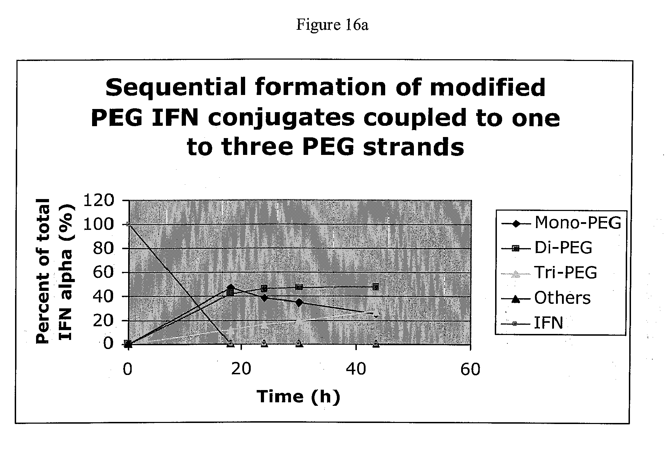

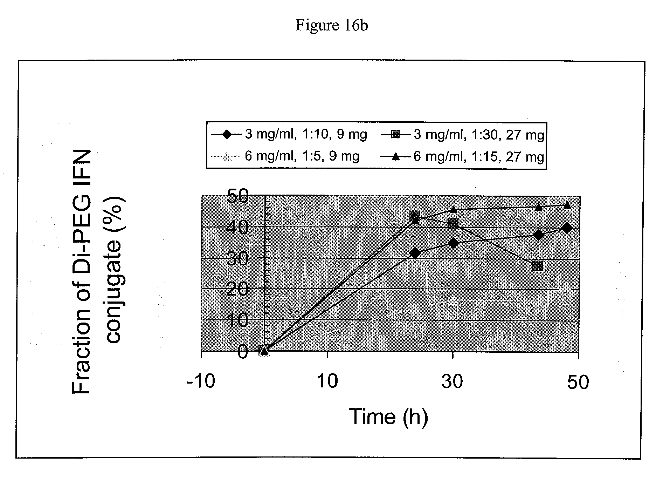

In general, converting recombinant human IFN α 2a, e.g. at a concentration of 6 mg/ml, with mPEG aldehyde in a 1:15 molar ratio to PEG IFN α 2a conjugates yielded predominantly dipegylated IFN α 2a conjugates from a reaction time of approximately 20 hours onwards (see figure 16a).

**Table 11: Relative percentage of differently pegylated IFN species at a 30 h reaction time with a single coupling cycle.**

| | Mono-PEG IFN (%) | Di-PEG IFN(%) | Tri-PEG IFN(%) |
|---|---|---|---|
| Percent of total IFN | 34.2 | 46.7 | 19.1 |

In another set of experiments, various amounts of solid mPEG aldehyde (from 3 until 30 mg) were added to different protein concentrations (3 mg/ml, 6 mg/ml) resulting in molar ratios of about 1:1,5, 1:3, 1:5, 1:10; 1:15; and 1:30 (protein:PEG). The reaction was performed at low temperature (5°C) as described under above and the samples were analysed as described above.

To achieve pre-dominantly di-PEG IFN alpha formation the molar ratio IFN:PEG had to be at least 1:15 which corresponds to a stoichiometric ratio of at least 1:1.25 of the number of available amino groups within the protein (in total 12 amino groups) to the number of PEG molecules.

The maximal product formation (~40% synthesis yield) was achieved after 20 hours incubation, but the kinetic of product formation could be influenced increased temperature (+ 20°C) and higher PEG amount (at least 2 times) (see figure 16b).

To increase the synthesis yield for di-pegylated IFN α 2a conjugates, it is recommendable to further optimize some process parameters such as, but not limited to, reaction pH, protein concentration, molar ratio protein: PEG, and possibly to introduce a re-cyclisation step to achieve a higher di-PEG protein conjugate yield, as described in the present invention under examples A 3 and A 4.

Besides the incubation time identified as an important parameter, the PEG concentration (expressed in molar ratio protein to PEG) in dependence to the protein concentration used is another important driving factor of the reaction towards pre-dominant di-PEG IFN α 2a product formation. Molar ratios of protein to PEG of =1:15 are directing the reactions towards di-PEGylation.

### 2. Preparative C-IEX purification

After the first pegylation reaction, di-pegylated IFN α 2a conjugates were separated from other IFN α 2a isoforms by preparative cation exchange chromatography as described in example A 5 applying the same linear salt gradient as used for C-IEX HPLC analytics. Each elution peak was analytically re-chromatographed (C-IEX HPLC) to identify the corresponding pegylated IFN α 2a isoforms (see above).

### Results

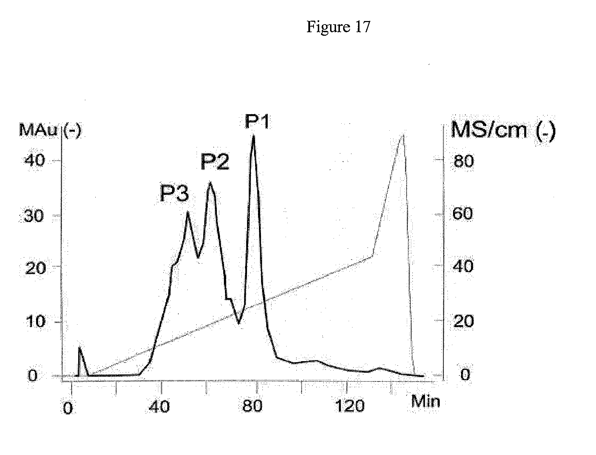

The linear gradient elution resulted in three major peaks which are shown in Figure 17 and summarized in Table 12.

**Table 12: Allocation of elution peaks from the preparative C-IEX run with corresponding retention time and allocated PEG IFN α 2a species (isoforms).**

| **Peak** | **No.3** | **No.2** | **No.1** |
|---|---|---|---|
| **Retention time (min)** | ~52 | ~61 | ~80 |
| **PEG IFN alpha 2a species containing** | Tri/Di | Di | Mono |

For di-pegylated IFN alpha 2a, one major di-PEG IFN α 2a peak could be identified. However, the shape of the peak lets assume, that potentially more than one out of 66 possible isoforms are eluting at the same time. A potential product for IFN would most likely consist of at least three isoforms because the corresponding peak revealed two shoulders.

### D) Dipegylation of glycosylated erythropoietin (EPO)

### 1. Pegylation Reaction and Analytics

12 kDa mPEG acetal was activated for the pegylation reaction as described in example A 1. Prior to the pegylation reaction, the sample of glycosylated EPO was dialysed against acidic acetate buffer and finally concentrated by ultrafiltration to approximately 4-8 mg/ml depending on the reaction conditions using centricon tubes (2 ml) equipped with YM 10 membranes of 10 kDa molecular weight cut off.

For EPO, an absorbance of 0.815 at 280 nm corresponds to 1 mg/ml protein. To calculate the measured protein concentration of pegylated EPO conjugates, the same molar extinction coefficient was applied.

6 mg solid mPEG aldehyde (molecular weight 12 kDa) were added to 6 mg/ml glycosylated EPO in 100 mM phosphate buffer, pH 7.5, resulting in a 1:5 molar ratio of EPO to mPEG aldehyde. 20 mM sodium cyanoborohydride was added and the reaction mixture was further incubated by gentle stirring at 5°C for several hours. The reaction was stopped by acidification with 8 N HCl.

The species distribution (mono-, di-, tri-) in the PEG EPO reaction mixture was determined by C-IEX HPLC. Therefore, the stopped reaction mixture was diluted three times with 40 mM acetate buffer (pH 3.8) and passed through a column packed with SP-5PW (75 x 7.5 mm, 10 µm particle size, 3.3 ml bed volume, manufactured by Tosohaas Bioscience), mounted on a Dionex system or Shimadzu HPLC system.

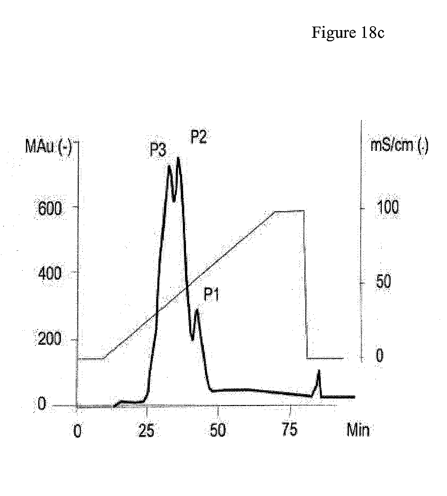

The column was equilibrated in 40 mM NaAc, pH 3.8 and 20% (v/v) isopropanol. The flow rate was 1 ml/min and typically 200 µg of protein were injected. After column washing, a linear gradient elution was done with 1 M NaCl in 40 mM NaAc, pH 3.8 containing 20% (v/v) isopropanol in approximately 20 column volume (see Figure 18c).

The column was run at room temperature; however, the samples were maintained at 5°C in the auto- injector.

For identification of the different pegylated species, the elution peaks of the analytical C-IEX HPLC were mass analysed (MALDI-TOF) and analysed by SDS PAGE, which allowed the allocation of each pegylated EPO peak to the retention time of an elution peak.

Combined analysis by mass spectrometry and SDS PAGE with coomassie (protein labelling) and iodine staining (PEG labelling) agreed with the preliminary mass estimation based on the amino acid sequence and PEG moiety (see Table 13).

**Table 13: Comparison of calculated and experimentally determined molecular weight of different pegylated EPO species.**

| **Molecular weight (estimated)** | **Molecular weight (MALDI-TOF)** | **Number of PEG molecules attached** | **Abbreviated name** |
|---|---|---|---|
| **[kDalton]** | **[kDalton]** | | |
| 34 | 27.9 | - | non-modified |
| | | | glycosylated EPO |
| 46 | 40.6 | 1 | Mono-PEG EPO |
| 58 | 52.9 | 2 | Di-PEG EPO |
| 70 | 63.5/65.6 | 3 | Tri-PEG EPO |

With the information from the mass analysis / SDS PAGE a retention time could be allocated to the corresponding PEG EPO species. The analytic C-IEX allowed the separation of mono-PEG -, di-PEG- and tri-PEG EPO isoforms in dependence of the retention time.

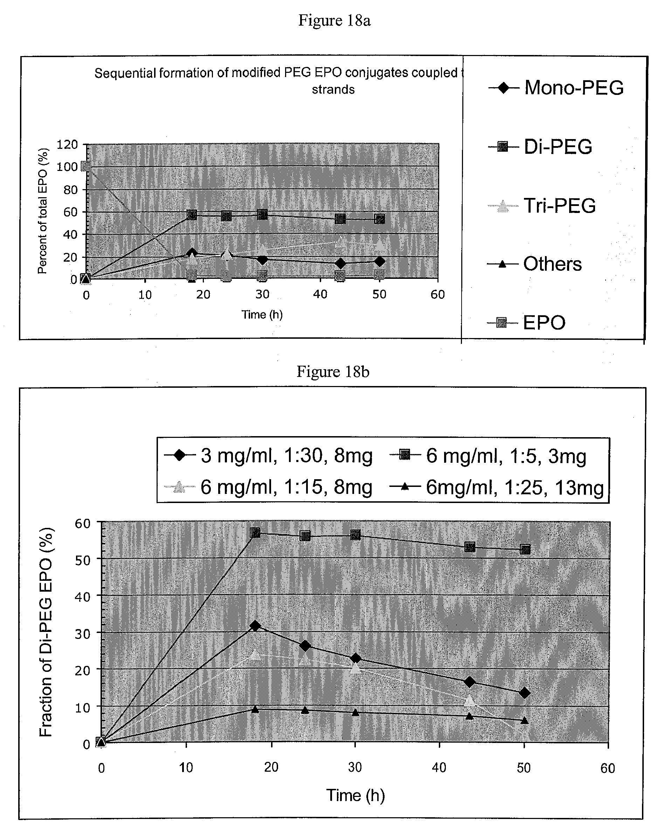

In general, converting recombinant human glycosylated EPO with mPEG aldehyde in a molar ratio of at least 1:5 (glycosylated EPO to PEG) yielded predominantly di-pegylated EPO conjugates from a reaction time of approximately 12 to 18 hours onwards (see figure 18a).

In another set of experiments, various amounts of solid mPEG aldehyde (from 3 to 13 mg) were added to different protein concentrations (3 mg/ml, 6 mg/ml) resulting in molar ratios of about 1:5; 1:15; 1:25 and 1:30 (protein:PEG). The reaction was performed at low temperature (5°C) as described under above and the samples were analysed as described above.

Interestingly, the effect of protein glycosylation probably causes a shielding effect on the surface of the protein, leading to selective PEG attachment. Thus, it is not surprising that dipegylation already occurs at a stoichiometric ratio of the number of amino groups within the protein to the number of PEG molecules of 1:0.5 (see Figure 18b).

To increase the synthesis yield for di-pegylated glycosylated EPO conjugates, it is recommendable to further optimize parameters such as, but not limited to, protein concentration and molar ratio protein:PEG and, possibly, to introduce a re-cyclisation step to achieve a higher di-PEG protein conjugate synthesis yield, as described in the present invention under examples A3 and A4.

Besides the incubation time identified as an important parameter, the PEG concentration (expressed in molar ratio protein to PEG, or better in molar excess to the available amino groups of the target protein for PEG attachment) in dependence to the protein concentration used is another important driving factor of the reaction towards pre-dominant di-PEG erythropoietin product formation. Molar ratios of target protein to PEG of about 1:5, or better a stoichiometric ratio of the number of amino groups within the protein and the number of PEG molecules of at least 1:0.5 are directing the reactions towards di-PEGylation

### E) Dipegylation of human growth hormone (hGH)

### 1. Pegylation reaction and Analytics

12 kDa mPEG acetal was activated for the pegylation reaction as described under example A 1. Prior to the pegylation reaction, the lyophilized human growth hormone sample was dissolved in water, dialysed against bicine buffer and finally concentrated by ultrafiltration to a concentration of approximately 4-8 mg/ml depending on the reaction conditions using centricon tubes (2 ml) equipped with YM 10 membranes of 10 kDa molecular weight cut off.

For hGH, an absorbance of 0.72 at 280 nm corresponds to 1 mg/ml protein. To calculate the measured protein concentration of pegylated hGH conjugates, the same molar extinction coefficient was applied.

25 mg solid mPEG aldehyde (molecular weight 12 kDa) were added to 6.0 mg/ml hGH in 100 mM bicine buffer, pH 7.5, resulting in a 1:15 molar ratio of hGH to mPEG aldehyde. 20 mM sodium cyanoborohydride were added, and the reaction mixture was further incubated by gentle stirring at 2-8°C for several hours. The reaction was stopped by acidification with 8 N HCl.

The species distribution (mono-, di-, tri-) in the PEG hGH reaction mixture was determined by C-IEX HPLC. Therefore, the stopped reaction mixture was diluted for three times with 40 mM NaAc buffer (pH 4.2) and passed through a column packed with SP-5PW (75 x 7.5 mm, 10 µm particle size, 3.3 ml bed volume, manufactured by Tosohaas Bioscience), mounted on a Dionex system or Shimadzu HPLC system.

The column was equilibrated with the same buffer, the flow rate was 0.4 ml/min and typically 200 µg of protein were injected. The column was run at room temperature; however, the samples were maintained at 5°C in the auto- injector.

For identification of the different pegylated species, the elution peaks of the analytical C-IEX HPLC were mass analysed by MALDI/TOF and SDS PAGE which allowed the allocation of each pegylated hGH peak to the retention time of an elution peak.

MALDI/TOF results agreed with the preliminary mass estimation based on the amino acid sequence and PEG moiety (see Table 14).

**Table 14: Comparison of calculated and experimentally determined molecular weight of different pegylated human growth hormone species.**

| **Molecular weight (estimated)** | **Molecular weight (SDS PAGE)** | **Number of PEG molecules attached** | **Abbreviated name** |
|---|---|---|---|
| **[kDalton]** | **[kDalton]** | | |
| 22 | n.d. | - | non-modified hGH |
| 34 | n.d. | 1 | Mono-PEG hGH |
| 46 | 47.4 | 2 | Di-PEG hGH |
| 58 | 59.0 | 3 | Tri-PEG hGH |

| | | | |
|---|---|---|---|
| n.d. - not determined | | | |

With the information from MALDI/TOF and SDS PAGE analysis a retention time could be allocated to the corresponding PEG hGH species. The analytical C-IEX allowed the separation of mono-PEG -, di-PEG- and tri-PEG hGH isoforms in dependence of the retention time.

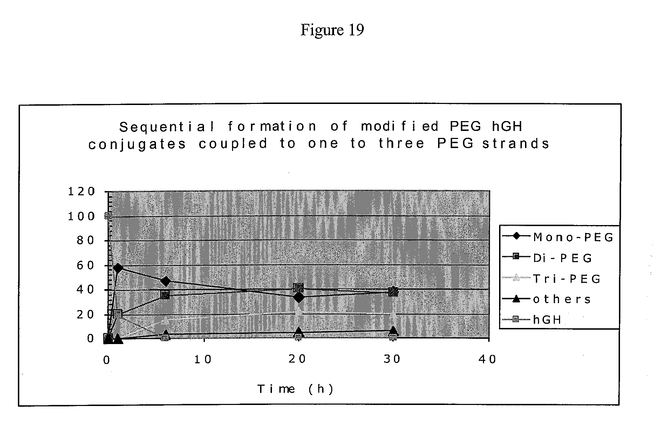

In general, converting recombinant human hGH with mPEG aldehyde at molar ratios of 1:15 to PEG-hGH conjugates yielded predominantly di-pegylated hGH conjugates from a reaction time of approximately 15 hours onwards (see Figure 19). The molar ratio of 1:15 corresponds to a stoichiometric ratio of the number of amino groups (nine) within the protein to the number of PEG molecules of 1:1.6.

Besides the incubation time identified as an important parameter, the PEG concentration used (expressed in molar ratio protein to PEG) is driving the reaction towards predominantly di-PEG hGH conjugate product formation. Molar ratios protein to PEG of = 1:10 are directing the reactions towards di-PEGylation.

### Figure legends

**Figure 1:** Overview of all process steps in the production of di-polymer protein conjugates according to the present invention.
**Figure 2:** Maximum yield of all di-PEG G-CSF conjugate isoforms and kinetic of formation as function of time-dependent low temperature storage of activated mPEG aldehyde used for the pegylation reaction.
**Figure 3a:** Sequential formation of recombinant G-CSF conjugates with one to four strands of 12 kDa mPEG aldehyde in time-dependent manner. Maximum di-PEG G-CSF yield at 30 h incubation.
**Figure 3b:** Kinetic of product formation of di-PEG G-CSF conjugate product being a mixture of two isoforms in dependence of the amounts of protein and PEG used. Various amounts of PEG and protein were tested according to the invention at pH 7.5, 5°C, gentle stirring in 0.5 mL reaction volume.
**Figure 3c:** Formation of di-PEG G-CSF conjugate mixture as function of PEG amount at a given incubation time (t=30h). pH (7.5), temperature (5°C), gentle stirring of 0.5 mL reaction volume were chosen according to the invention.
**Figure 3d:** Influence of temperature (22°C versus 5°C) on the kinetic of product formation, di-PEG G-CSF conjugate mixture, during reaction (5.7 mg/ml protein, molar ratios as indicated in 0.5 mL reaction volume).
**Figure 4:** A typical preparative C-IEX elution profile for the separation of di-PEG G-CSF from the pegylation reaction mixture (protein load: 6.1 mg pegylated G-CSF species; column: SP-5PW C-IEX column (Tosohaas, 20 µm, 10.6 ml bed volume), Buffer A: 15mM NaAc, pH 4.2; Buffer B: 1 M NaCl in 15 mM NaAc, pH 4.2; linear gradient); E- eluat, RP - rest pool.
**Figure 5:** C-IEX HPLC separation of Di-PEG G-CSF product. Chromatogramm recorded at 214 nm. Isoforms are labelled.
**Figure 6:** SEC separation of di-PEG G-CSF product. Chromatogramm recorded at 214 nm. Peak at 23 min corresponds to salt.
**Figure 7:** CD spectrum of non-modified G-CSF (black), and di-PEG G-CSF product (red).
**Figure 8:** *In vivo* bioactivity of the di-PEG G-CSF conjugate product comprising a mixture (0.76:1) of di- and di'-PEG G-CSF conjugate isoforms in comparison to unmodified rhG-CSF (Neupogen ®) and mono-pegylated rhG-CSF (Neulasta®) illustrated by the average WBC after single subcutaneous injection (PBS - phosphate buffered saline).
**Figure 9:** *In vivo* bioactivity of the di-PEG G-CSF conjugate product comprising a mixture (0.76:1) of di- and di'-PEG G-CSF conjugate isoforms in comparison to unmodified rhG-CSF (Neupogen ®) and mono-pegylated rhG-CSF (Neulasta®) illustrated by the average neutrophil counts after single subcutaneous injection (PBS - phosphate buffered saline).
**Figure 10:** *In vivo* bioactivities of the di-PEG G-CSF isoform and the di'-PEG G-CSF isoform in comparison to to di-PEG G-CSF conjugate product being a mixture of both isoforms (0.76:1) illustrated by the average WBC after single subcutaneous injection (PBS - phosphate buffered saline).
**Figure 11:** In vivo bioactivities of di-PEG G-CSF isoform and di'-PEG G-CSF isoform in comparison to di-PEG G-CSF conjugate product being a mixture of both isoforms (0.76:1) illustrated by the average neutrophil counts after single subcutaneous injection (PBS - phosphate buffered saline).
**Figure 12:** Analytical C-IEX elution profiles of reaction mixtures after 30h pegylation of G-CSF (3.2 mg/ml) with mPEG 12kDa (molar ratio 1:25, upper chromatogram) and with mPEG 20kDa (molar ratio 1:19, lower chromatogram) analysed on a Dionex system equipped with a SP 5PW column under identical conditions (UV 214 nm). The peak profiles are matching, which allows i) the peak identification and ii) peak allocation to the corresponding pegylated G-CSF species (mono- di- and di' isoforms).
**Figure 13:** Sequential formation of recombinant G-CSF conjugates with one to four strands of 20kDa mPEG aldehyde in time-dependent manner. Predominantly di-PEG G-CSF yield is achieved from 18h incubation onwards. The reaction conditions were 3.2 mg/ml G-CSF with 1:19 molar ratio G-CSF to PEG 20k, 5°C, gentle stirring.
**Figure 14:** *In vivo* bioactivities of Di'-PEG 20k G-CSF isoform in comparison to unmodified rhG-CSF (Neupogen®) and mono-pegylated rhG-CSF (Neulasta®) illustrated by the average WBC after single subcutaneous injection (PBS - phosphate buffered saline). **Figure 15:** *In vivo* bioactivities of Di'-PEG 20kDa G-CSF isoform in comparison to unmodified rhG-CSF (Neupogen®) and mono-pegylated rhG-CSF (Neulasta®) illustrated by the average neutrophil counts after single subcutaneous injection (PBS - phosphate buffered saline).
**Figure 16a:** Sequential formation of recombinant IFN alpha 2a conjugates with one to three strands of 12 kDa mPEG aldehyde in time-dependent manner. Pre-dominantly di-PEG IFN alpha 2a yield is already achieved after approximately 20h incubation, and lasts over the whole investigated time frame of 50 hours.
**Figure 16b:** Kinetic of product formation of di-PEG IFN α 2a conjugate product in dependence of the amounts of protein and PEG used. Various amounts of PEG and protein were tested according to the invention at pH 7.5, 5°C, gentle stirring in 0.5 mL reaction volume.
**Figure 17:** A preparative C-IEX elution profile for the separation of di-PEG IFN alpha 2a conjugate from the pegylation reaction mixture (protein load: 6 mg pegylated IFN alpha 2a after 24h incubation; column: SP-5PW C-IEX column (Tosohaas, 20 µm, 10.6 ml bed volume), Buffer A: 40mM NaAc, pH 4.2; Buffer B: 1 M NaCl in 40 mM NaAc, pH 4.2; linear gradient); P1 - Mono-PEG; P2 - Di-PEG; P3 - Mixture Tri-./ Di-PEG.
**Figure 18a:** Sequential formation of recombinant EPO conjugates with one to three strands of 12 kDa mPEG aldehyde in time-dependent manner. Predominantly di-PEG EPO yield is surprisingly achieved within the first 18h of incubation and lasts for the rest of the incubation
**Figure 18b:** Kinetic of product formation of di-PEG erythropoietin conjugate product in dependence of the amounts of protein and PEG used. Various amounts of PEG and protein were tested according to the invention at pH 7.5, 5°C, gentle stirring in 0.5 mL reaction volume.
**Figure 18c:** Analytical C-IEX elution profile for the separation of di-PEG EPO conjugate from the pegylation reaction mixture (protein load: 1 mg pegylated EPO mixture after 24h incubation; column: SP-5PW C-IEX column (Tosohaas, 10 µm, 3.3 ml bed volume), Buffer A: 40mM NaAc, pH 3.8, 20 % Isoporpanol; Buffer B: 1 M NaCl in 40 mM NaAc, pH 3.8; 20% Isopropanol, linear gradient); P1 - Mono-PEG; P2 - Di-PEG; P3 - Tri-PEG
**Figure 19:** Sequential formation of recombinant hGH conjugates with one to three strands of 12 kDa mPEG aldehyde in time-dependent manner. Pre-dominantly di-PEG hGH yield is achieved after approximately 15h incubation, and lasts.

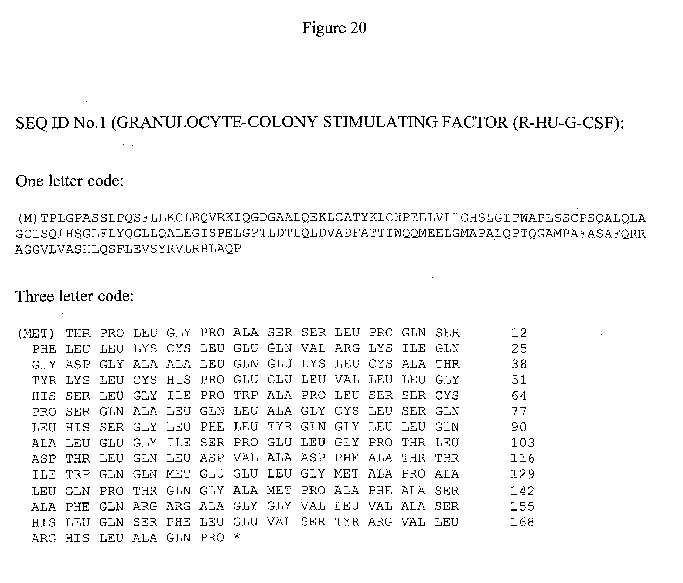

**Figure 20:** SEQ ID No. 1 (amino acid sequence of G-CSF including methionine at position 1).
**Figure 21:** SEQ ID No. 2 (amino acid sequence of IFN a 2a)
**Figure 22:** SEQ ID No. 3 (amino acid sequence of EPO)
**Figure 23:** SEQ ID No. 4 (amino acid sequence of hGH)

## Claims

1. Polymer-protein conjugate, wherein two nitrogen atoms of amino groups of the protein are each conjugated with a polymer unit via an amine linkage.

2. The conjugate according to claim 1,
wherein the polymer unit comprises at least one polymer moiety and a linker moiety which is located between the at least one polymer moiety and the amine linkage.

3. The conjugate according to claim 2,
wherein the linker moiety is linear.

4. The conjugate according to claim 2,
wherein the linker moiety is branched.

5. The conjugate according to claim 4,
wherein a polymer unit comprises more than one polymer moiety.

6. The conjugate according to any of the preceding claims,
wherein the linker moiety comprises at least one methylene group attached to the nitrogen atom of the amine linkage.

7. The conjugate according to claim 6,
wherein the linker moiety comprises from 1 to 12, preferably from 1 to 5, more preferably from 1 to 3 and most preferably two methylene groups attached to the nitrogen atom of the amine linkage.

8. The conjugate according to any of the preceding claims,
wherein the protein is a biologically active protein.

9. The conjugate according to any of the preceding claims,
wherein the protein is glycosylated or non-glycosylated.

10. The conjugate according to claim 9,
wherein the protein has a glycosylation pattern which corresponds to that of the protein in a naturally-occuring form.

11. The conjugate according to claim 9,
wherein the protein has a glycosylation pattern which differs from that of the protein in a naturally-occuring form.

12. The conjugate according to any of the preceding claims, having the formula:
[(R-L¹-Polymer)_{y}-L²-(CH₂-)ₘ-NH]₂-P
wherein R is H, lower alkyl, aryl or any suitable protecting group; Polymer is a polymer which is suitable to be conjugated with P; m is an integer representing the number of methylene groups, P is a biologically active protein or proteinoid wherein two nitrogen atoms of amino groups of the protein or proteinoid are each conjugated with a polymer unit, L¹ is O, N, S and/or a branched or non-branched linker moiety which can be absent or present; L² is a branched or non-branched linker moiety which can be absent or present; and y is a integer with the proviso that y is 1 in the absence of L² and y is at least 1 in the presence of L².

13. The conjugate according to any of the preceding claims,
wherein the polymer is selected from the group consisting of polyalkylene glycol, polysaccharide, pyrrolidone, cellulose, polyaminoacids, polyacrylamide and derivatives thereof.

14. The conjugate according to any of the preceding claims, having the formula:
[(R-L¹-PEG)_{y}-L²-(CH₂-)ₘ-NH]₂-P
wherein R is H, lower alkyl, aryl or any suitable protecting group; PEG is a polyethylene glycol moiety; m is an integer representing the number of methylene groups, P is a biologically active protein or proteinoid wherein two nitrogen atoms of amino groups of the protein or proteinoid are each conjugated with a polyethylene glycol unit, L¹ is O, N, S and/or a branched or non-branched linker moiety which can be absent or present; L² is a branched or non-branched linker moiety which can be absent or present; and y is a integer with the proviso that y is 1 in the absence of L² and y is at least 1 in the presence of L².

15. The conjugate according to claim 14,
wherein PEG has the formula -(CH₂-CH₂-O)ₙ with n representing the number of ethylene oxide residues in the polyethylene glycol unit.

16. The conjugate according to claim 14 or 15,
wherein y is 1.

17. The conjugate according to any of the preceding claims,
wherein the amino groups are selected from the group consisting of the N-terminal amino group, ε-amino groups, guanidinium groups and imidazole groups.

18. The conjugate according to any of the preceding claims,
wherein the protein or proteinoid has the biological activity of erythropoietin, interferon alpha, interferon beta, interferon gamma, consensus interferon, human growth hormone, and/or structural and/or functional variants and/or fragments thereof.

19. The conjugate according to claim 18,
wherein the protein or proteinoid has the biological activity of IFN a 2a.

20. The conjugate according to claim 18,
wherein the protein or proteinoid has the biological activity of erythropoietin.

21. The conjugate according to claim 18,
wherein the protein or proteinoid has the biological activity of human growth hormone.

22. The conjugate according to any of the preceding claims,
wherein the two amino groups are selected from the group consisting of the N-terminal amino group and ε-amino groups of lysine residues.

23. The conjugate according to any of the preceding claims,
wherein the protein has an amino acid sequence which corresponds to the wild-type sequence of the protein in that amino acids of the wild-type sequence containing amino groups are neither deleted nor modified nor added.

24. The conjugate according to any of claims 18, 19, 22 or 23,
wherein the IFN a 2a has the amino acid sequence identified in SEQ ID No.2.

25. The conjugate according to any of claims 18, 20, 22 or 23,
wherein the erythropoietin has the amino acid sequence identified in SEQ ID No.3.

26. The conjugate according to claim 27,
wherein the erythropoietin is glycosylated.

27. The conjugate according to any of claims 18, 21, 22 or 23,
wherein the human growth hormone has the amino acid sequence identified in SEQ ID No.4.

28. The conjugate according to claim 12 or 14,
wherein R is a lower alkyl or aryl selected from the group consisting of methyl, ethyl, propyl, butyl and benzyl.

29. The conjugate according to any of claims 13 to 28,
wherein the molecular weight of a polyethylene glycol moiety is from 2 to 100 kD, more preferably from 5 to 60 kD and most preferably from 10 to 30 kD.

30. The conjugate according to of claims 12 to 29,
wherein m is from 1 to 12, preferably from 1 to 5, more preferably from 1 to 3 and most preferably is 2.

31. Pharmaceutical preparation comprising at least one and preferably two polymer-protein conjugates according to one of claims 1 to 30.

32. A mixed pharmaceutical preparation of PEG protein conjugates comprising at least one di-pegylated protein conjugate according to one of claims 14 to 30 mixed with multi-pegylated protein conjugates wherein the proportion of said di-pegylated protein conjugate in the mixed pharmaceutical preparation is pre-determined.

33. The preparation according to any of claims 31 to 32,
further comprising a pharmaceutically acceptable diluent, carrier, buffer and/or adjuvant.

34. Process for the preparation of a polymer-protein conjugate,
which comprises a first reaction step in which a protein is reacted with a polymer reagent to yield polymer protein conjugates, and a recycling step in which the reaction step is repeated with a part or all of non-desired products of the first reaction step.

35. Process for the preparation of a polymer-protein conjugate,
wherein the process comprises reacting a protein having at least two amino groups with a polymer reagent having a single aldehyde group in the presence of a reducing agent and at a reaction time being adjusted to selectively yield polymer protein conjugates having two nitrogen atoms of amino groups of the protein conjugated with a polymer unit via an amine linkage.

36. Process according to claim 34 or 35,
wherein the polymer reagent comprises a polyalkylene glycol, polysaccharide, pyrrolidone, cellulose, polyaminoacid moiety, and/or polyacrylamide and derivatives thereof.

37. Process according to any of claims 34 to 36,
wherein the protein is a protein or proteinoid having the biological activity of erythropoietin, interferon alpha, interferon beta, interferon gamma, consensus interferon, human growth hormone, and/or structural and/or functional variants and/or fragments thereof.

38. Process according to any of claims 34 to 37,
wherein the reaction time is chosen such that at least 20%, preferably at least 25%, more preferably at least 30% and most preferably at least 40% of the process products are polymer-protein conjugates having two nitrogen atoms of amino groups of the protein conjugated with a polymer unit via an amine linkage.

39. The process according to any of claims 34 to 38,
wherein the reaction time is from 6 h to 48 h and preferably from 12 h to 48 h and more preferably from 16 h to 36 h and most preferably from 18 h to 32 h.

40. The process according to any of claims 34 to 39,
wherein the polymer-protein conjugate is a polymer-protein conjugate according to any of claims 1 to 30.

41. The process according to any of claims 34 to 40,
wherein the reaction is performed at a protein concentration from 0.5 to 100 mg/ml, more preferably from 1 to 10 mg/ml, and most preferably at about 3.2 mg/ml.

42. The process according to any of claims 34 to 41,
wherein the reaction is performed at a protein to polymer molar ratio of from 1:1 to 1:400 and preferably from 1:5 to 1:50, and more preferably from 1:10 to 1:30.

43. The process according to any of claims 34 to 42,
wherein, if the protein is a glycosylated protein, the reaction is performed at a stoichiometric ratio of amino groups within the protein to polymer molecules of from 10:1 to 1:80 and preferably from 1:0.3 to 1:50 and more preferably from 2:1 to 1:20.

44. The process according to any of claims 34 to 42,
wherein, if the protein is a non-glycosylated protein, the reaction is performed at a stoichiometric ratio of amino groups within the protein to polymer molecules of from 1:1 to 1:80, preferably from 1:1.25 to 1:80, more preferably from 1:1.25 to 1:50 and even more preferably from 1:1.25 to 1:30 and most preferably from 1:2 to 1:10.

45. The process according to one of claims 34 to 44,
wherein the reducing agent used in the reductive alkylation is NaCNBH₄ and/or NaBH₄.

46. The process according to one of claims 34 to 45,
wherein the reaction is performed at a pH from 6.9 to 7.8.

47. The process according to one of claims 34 to 46,
wherein the reaction is performed in the presence of a buffer selected from phosphate, acetate, HEPES, MES, and/or other suitable buffers.

48. The process according to any of claims 34 to 47,
wherein the reaction is performed at a temperature from 2°C to 50°C, preferably at a temperature from 2°C to 8°C.

49. The process according to any of claims 34 to 48,
wherein the polymer reagent is a reagent having the formula
[R-L¹-(CH₂-CH₂-O)ₙ]_{y}-L²-(CH₂-)ₘ₋₁-CHO
wherein R is H, a lower alkyl, aryl or any suitable protecting group; n is an integer representing the number of ethylene oxide residues in a polyethylene glycol moiety; m is an integer representing the number of methylene groups; L¹ is O, N, S and/or a branched or non-branched linker moiety which can be absent or present; L² is a branched or non-branched linker moiety which can be absent or present; and y is a integer with the proviso that y is 1 in the absence of L² and y is at least 1 in the presence of L².

50. The process according to claim 49,
wherein the polymer reagent is a PEG acetaldehyde, preferably methoxy polyethylene glycol acetaldehyde.

51. The process according to claim 50,
wherein the methoxy polyethylene glycol acetaldehyde is stored as a solid substance under nitrogen and at a temperature of -20°C or less.

52. The process according to claim 50 or 51,
wherein the polyethylene glycol acetaldehyde is obtained by activating a corresponding polyethylene glycol diethyl acetal.

53. The process according to one of claims 34 to 52,
further comprising a purification step leading to a product pool of di-polymer-protein conjugates wherein two defined amino groups of the protein are each conjugated with a polymer unit, and to a rest pool which contains unreacted protein, unreacted polymer reagent, di-polymer-protein conjugate isoforms other than those of the product pool and/or polymer-protein conjugates with less or more than two amino groups of the protein conjugated with a polymer unit.

54. The process according to claim 53,
wherein the purification step is performed by ion exchange chromatography.

55. The process according to claim 53 or 54,
wherein the process according to claims 34 to 52 is repeated with the rest pool and the resulting product pool is preferably combined with the product pool.

56. The process according to claim 34,
wherein the first reaction step corresponds to the process according to one of claims 34 to 52.

57. The process according to claims 34,
wherein the recycling step comprises the process according to claim 55.

58. Polymer-protein conjugates prepared according to the process of any of claims 34 to 57.

59. Use of a polymer-protein conjugate according to any of claims 1 to 30 or 58 or of a pharmaceutical preparation according to any of claims 31 to 33 for the manufacture of a medicament for the treatment of a disorder.

60. The use according to claim 59,
wherein the polymer-protein conjugate is a polymer-IFN a 2a conjugate.

61. The use according to claim 60,
wherein the disorder is an infectious disease, in particular hepatitis.

62. The use according to claim 59,
wherein the polymer-protein conjugate is a polymer-erythropoietin conjugate.

63. The use according to claim 62,
wherein the disorder is an anemia.

64. The use according to claim 59,
wherein the polymer-protein conjugate is a polymer- growth hormone conjugate.

65. The use according to claim 64,
wherein the disorder is a growth hormone deficiency.

66. Use of a polymer-protein conjugate according to any of claims 1 to 30 or 58 for reducing immunogenicity of a protein.


### Annex to the application documents - subsequently filed sequences listing

SEQUENCE LISTING

<110>  SIEGFRIED Ltd

<120>  Di-polymer protein conjugates and processes for their preparation

<130>  S 7865

<140>  EP06111062.3
<141>  2006-03-13

<160>  7

<170>  PatentIn version 3.3

<210>  1
<211>  175
<212>  PRT
<213>  human (r-hu-G-CSF)

<400>  1

Met Thr Pro Leu Gly Pro Ala Ser Ser Leu Pro Gln Ser Phe Leu Leu
1               5                   10                  15

Lys Cys Leu Glu Gln Val Arg Lys Ile Gln Gly Asp Gly Ala Ala Leu
            20                  25                  30

Gln Glu Lys Leu Cys Ala Thr Tyr Lys Leu Cys His Pro Glu Glu Leu
        35                  40                  45

Val Leu Leu Gly His Ser Leu Gly Ile Pro Trp Ala Pro Leu Ser Ser
    50                  55                  60

Cys Pro Ser Gln Ala Leu Gln Leu Ala Gly Cys Leu Ser Gln Leu His
65                  70                  75                  80

Ser Gly Leu Phe Leu Tyr Gln Gly Leu Leu Gln Ala Leu Glu Gly Ile
                85                  90                  95

Ser Pro Glu Leu Gly Pro Thr Leu Asp Thr Leu Gln Leu Asp Val Ala
            100                 105                 110

Asp Phe Ala Thr Thr Ile Trp Gln Gln Met Glu Glu Leu Gly Met Ala
        115                 120                 125

Pro Ala Leu Gln Pro Thr Gln Gly Ala Met Pro Ala Phe Ala Ser Ala
    130                 135                 140

Phe Gln Arg Arg Ala Gly Gly Val Leu Val Ala Ser His Leu Gln Ser
145                 150                 155                 160

Phe Leu Glu Val Ser Tyr Arg Val Leu Arg His Leu Ala Gln Pro
                165                 170                 175

<210>  2
<211>  165

<212>   PRT
<213>   human (IFN alpha 2a; mature sequence without leader sequence)

<400>   2

Cys Asp Leu Pro Gln Thr His Ser Leu Gly Ser Arg Arg Thr Leu Met
1               5                   10                  15

Leu Leu Ala Gln Met Arg Lys Ile Ser Leu Phe Ser Cys Leu Lys Asp
            20                  25                  30

Arg His Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Leu Ser Thr Asn Leu Gln Glu Ser
145                 150                 155                 160

Leu Arg Ser Lys Glu
                165


<210>   3
<211>   166
<212>   PRT
<213>   human (r-EPO; mature sequence without leader sequence)

<400>   3

Ala Pro Pro Arg Leu Ile Cys Asp Ser Arg Val Leu Gln Arg Tyr Leu
1               5                   10                  15

Leu Glu Ala Lys Glu Ala Glu Asn Ile Thr Thr Gly Cys Ala Glu His
            20                  25                  30

Cys Ser Leu Asn Glu Asn Ile Thr Val Pro Asp Thr Lys Val Asn Phe
        35                  40                  45

Tyr Ala Trp Lys Arg Met Glu Val Gly Gln Gln Ala Val Glu Val Trp
    50                  55                  60

Gln Gly Leu Ala Leu Leu Ser Glu Ala Val Leu Arg Gly Gln Ala Leu
65                  70                  75                  80

Leu Val Asn Ser Ser Gln Pro Trp Glu Pro Leu Gln Leu His Val Asp
                85                  90                  95

Lys Ala Val Ser Gly Leu Arg Ser Leu Thr Thr Leu Leu Arg Ala Leu
            100                 105                 110

Gly Ala Gln Lys Glu Ala Ile Ser Pro Pro Asp Ala Ala Ser Ala Ala
        115                 120                 125

Pro Leu Arg Thr Ile Thr Ala Asp Thr Phe Arg Lys Leu Phe Arg Val
    130                 135                 140

Tyr Ser Asn Phe Leu Arg Gly Lys Leu Lys Leu Tyr Thr Gly Glu Ala
145                 150                 155                 160

Cys Arg Thr Gly Asp Arg
                165

<210>  4
<211>  191
<212>  PRT
<213>  human (r-growth hormone; mature sequence without leader sequence)

<400>  4

<210>  5
<211>  14
<212>  PRT
<213>  Artificial

<220>
<223>  pegylated peptide; PEG attached to the alpha-amino-group of the
       N-terminal methionine; sequence from amino acid 1 to 13

<400>  5

Met Thr Pro Leu Gly Pro Ala Ser Ser Leu Pro Gln Ser Phe
1               5                   10

<210>  6
<211>  26
<212>  PRT
<213>  Artificial

<220>
<223>  pegylated peptide; PEG attached to the epsilon-amino-group of
       lysine 17; sequence from amino acid 14 to 40

<400>  6

Leu Leu Lys Cys Leu Glu Gln Val Arg Lys Ile Gln Gly Asp Gly Ala
1               5                   10                  15

Ala Leu Gln Glu Lys Leu Cys Ala Thr Tyr
            20                  25

<210>  7
<211>  26
<212>  PRT
<213>  Artificial

<220>
<223>  pegylated peptide; PEG attached to the epsilon-amino-group of
       lysine 35; sequence from amino acid 14 to 40

<400>  7

Leu Leu Lys Cys Leu Glu Gln Val Arg Lys Ile Gln Gly Asp Gly Ala
1               5                   10                  15

Ala Leu Gln Glu Lys Leu Cys Ala Thr Tyr
            20                  25